# EUROPEAN PATENT SPECIFICATION

(11) **EP 1 494 728 B1**
(45) Date of publication and mention of the grant of the patent: **10.03.2010**
(21) Application number: 03718375.3
(22) Date of filing: 10.04.2003
(51) Int. Cl.: A61L 26/00

(54) **TISSUE DEFECT DRESSINGS COMPRISING A KERATIN NETWORK**
KERATIN-NETZWERKE ENTHALTENDE WUNDVERBÄNDE FÜR GEWEBEDEFEKTE
PANSEMENTS POUR ANOMALIE TISSULAIRE COMPRENANT DES RESEAUX PROTEIQUES

(30) Priority: 10.04.2002 US 119477; 22.04.2002 US 127523; 26.04.2002 US 133885; 05.07.2002 US 393958 P
(43) Date of publication of application: 12.01.2005
(73) Proprietor: Keraplast Technologies Ltd., San Antonio, TX 78258 (US)
(72) Inventor: VAN DYKE, Mark, E., Fair Oaks Ranch, TX 78015 (US)
(74) Representative: Glawe, Delfs, Moll
(86) International application number: PCT/US2003/011364
(87) International publication number: WO 2003/086491

(56) References cited:
- EP-A- 0 097 907
- US-A- 5 412 076
- US-A- 5 948 432
- US-A- 5 955 549
- US-A- 6 124 265
- US-A- 6 159 496
- US-A- 6 165 496
- YOSHIO TANAKA ET AL: "REACTION OF WOOL KERATIN WITH EPOXIDES" PROCEEDINGS INTERNATIONAL WOLLTEXTIL-FORSCHUNGSKONFERENCE, XX, XX, vol. 3, 1976, pages 192-201, XP001154423

## Description

### Field of the Application

The present application relates to tissue defect dressings and to methods for their use. The tissue defect dressings comprise a proteinaceous salve, preferably a proteinaceous film or hydrogel, most preferably a keratinaceous film or hydrogel on a suitable support. Keratinaceous salves are biologically active and encourage correction of tissue defects due to growth factors which stimulate cell growth.

### Background of the Invention

A variety of wound dressings are known from the prior art, e.g. US-A-6 124 265 and US-A-5 412 076. US-A-6 124 265 discloses methods for producing thin keratin films which may be used as a wound dressing. US-A-5 412 076 describes collagen products which may be used as dressings in the form of films or felt.

A variety of wound healing preparations are available on the market. Desirable properties for wound healing preparations include the ability to isolate and protect wounds from invasion by infectious agents. A moist dressing is often beneficial. Some of the advantages of a moist wound dressing are: the rehydration of dehydrated tissue; increased angiogenesis, i.e., proliferation of new blood vessels; minimized bacterial growth; physical protection; and the maintenance of the proper pH for stimulating the release of oxygen and for allowing proteolytic enzymes to work more efficiently.

Available wound dressings have certain disadvantages. Powder or granules cannot be applied evenly, do not absorb tissue moisture evenly, and generally are difficult to remove completely from the wound bed. Pastes must be spread onto the tissue. The pressure required to spread the paste can be painful or further traumatize the tissue. In addition, an even application is not always easy to achieve because the product retains its plastic character.

Moist, biocompatible dressings are needed that are easy to apply, easy to maintain in place, easy to remove, and preferably contain factors that actually speed the rate and quality of healing.

### Summary of the Invention

The present invention is defined in the claims and provides a tissue defect dressing comprising a keratinaceous salve comprising keratinaceous molecules networked by interprotein associations consisting essentially of other than disulfide crosslinks. In a preferred aspect, the keratinaceous salve is selected from the group consisting of a keratinaceous hydrogel and a keratinaceous film.

Where the proteinaceous salve is a hydrogel, the hydrogel comprises water retained in a network comprising keratins comprising interkeratin associations consisting essentially of other than disulfide crosslinks. The interkeratin associations are selected from the group consisting of entanglements, electrostatic bonds, covalent bonds consisting essentially of other than disulfide bonds, and combinations thereof. In a preferred embodiment, the hydrogel comprises water retained in a network consisting essentially of water soluble keratins comprising covalent bonds consisting essentially of other than disulfide bonds, said covalent bonds consisting essentially of interprotein crosslinks comprising first covalent bonds between first functional groups on a plurality of molecules of a crosslinking agent and first reactive pendant groups on a plurality of first water soluble keratins and second covalent bonds between second functional groups on a plurality of molecules of said crosslinking agent and second reactive pendant groups on a plurality of second water soluble keratins.

Where the proteinaceous salve is a film, the film comprises water-soluble proteins consisting essentially of interprotein crosslinks comprising first covalent bonds between first functional groups on a plurality of molecules of a heterogeneous crosslinking agent and first reactive pendant groups on a plurality of first water soluble proteins and second covalent bonds between second functional groups on a plurality of molecules of said heterogeneous crosslinking agent and second reactive pendant groups on a plurality of second water soluble proteins.

The first and second functional groups preferably are selected from the group consisting of alkoxide groups, allyl groups, vinyl groups, hydroxyl groups, amine groups, aldehyde groups, isocyanate groups, ester groups, and anhydride groups. Preferably, the reactive pendant groups are selected from the group consisting of hydroxyl groups, thiol groups, reactive amine groups, and epoxides. Most preferably, at least one of a moiety selected from the group consisting of said first functional groups, said first reactive pendant groups, said second functional groups, and said second reactive pendant groups is an epoxide.

In a preferred embodiment, the keratins are derived from human hair. Keratinaceous tissue defect dressings inherently comprise and deliver bioactive components to the tissue defect. The tissue defect dressing also may comprise one or more added bioactive component(s).

The tissue defect dressing preferably further comprises a support adapted to support the proteinaceous salve. The support preferably comprises a sheet of permeable material having an inner surface abutting said proteinaceous salve and an outer surface abutting a barrier material.

The cohesive tissue defect dressing comprises a proteinaceous salve comprising a network comprising water soluble keratins comprising inter keratin crosslinks having the following general structure: 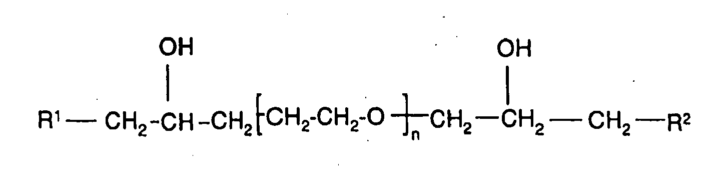 wherein
n is from 1 to 50; and,
R¹ and R² independently are amino acid residues of separate water soluble keratins, the residues being selected from the group consisting of cysteine, arginine, serine, lysine, asparagine, glutamine, tyrosine, tryptophan, and histidine.

In another aspect, the cohesive-tissue defect dressing comprises a proteinaceous salve comprising a network comprising water soluble keratins comprising interkeratin crosslinks having the following general structure: 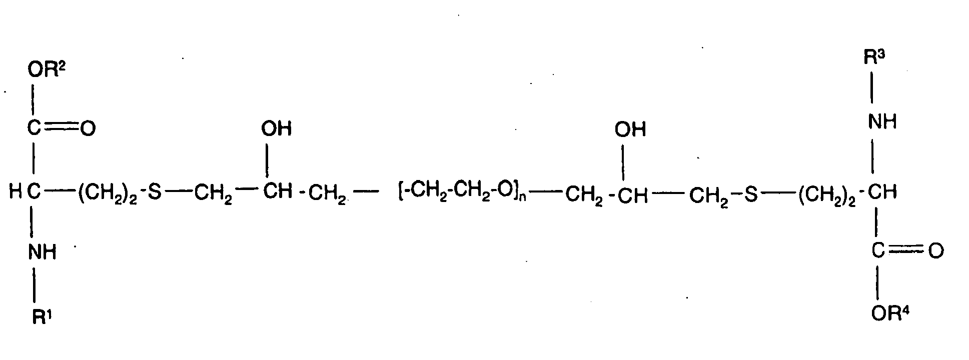 wherein
n is from 1 to 50;
R¹ and R² are the remainder of a first water soluble keratin; and,
R³ and R⁴ are the remainder of a second water soluble keratin.

### Brief Description of the Figures

Fig. 1 is an amino acid analysis of reductively extracted low molecular weight keratins, specifically sample 4-AKR-112-2A from Example 3.
Fig. 2 is a cone and plate viscosity analysis of keratin hydrogel made with 10 weight percent of sample no. 4-AKR-112-2B from Example 5.
Fig. 3 is a graph of the storage (G') and loss (G") dynamic shear moduli of a keratin hydrogel made with 10 weight percent of sample no. 4-AKR-112-2B from Example 5.
Fig. 4 is a graph of the dynamic shear viscosity of non-crosslinked keratin hydrogels from Example 5.
Fig. 5 is a graph of the dynamic shear viscosity of DER 332 resin crosslinked keratin hydrogels of Example 5.
Fig. 6 is a graph of the dynamic shear viscosity of three crosslinked keratin hydrogels of Example 5.
Fig. 7 is a graph of the tan d measurement of non-crosslinked and DER 332 resin crosslinked keratin hydrogels of Example 5.
Fig. 8 is a photograph from the *in vitro* scratch assay used in wound studies.
Fig. 9 is an illustration of the wound placement pattern in the porcine model.

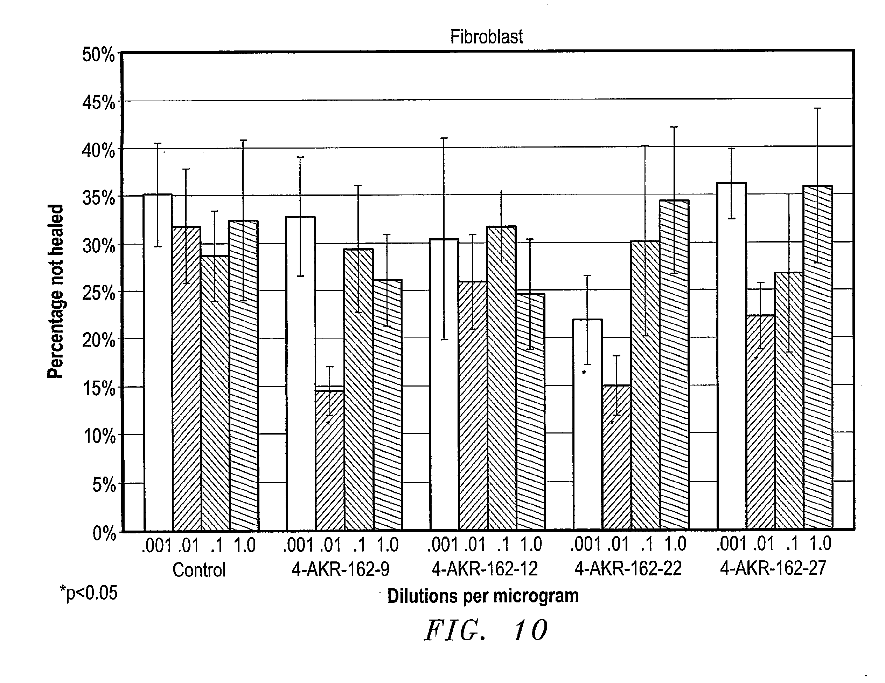

Fig. 10 is a chart of the percentage not healed vs. dilutions per milligram from the fibroblast cultures of Example 14.

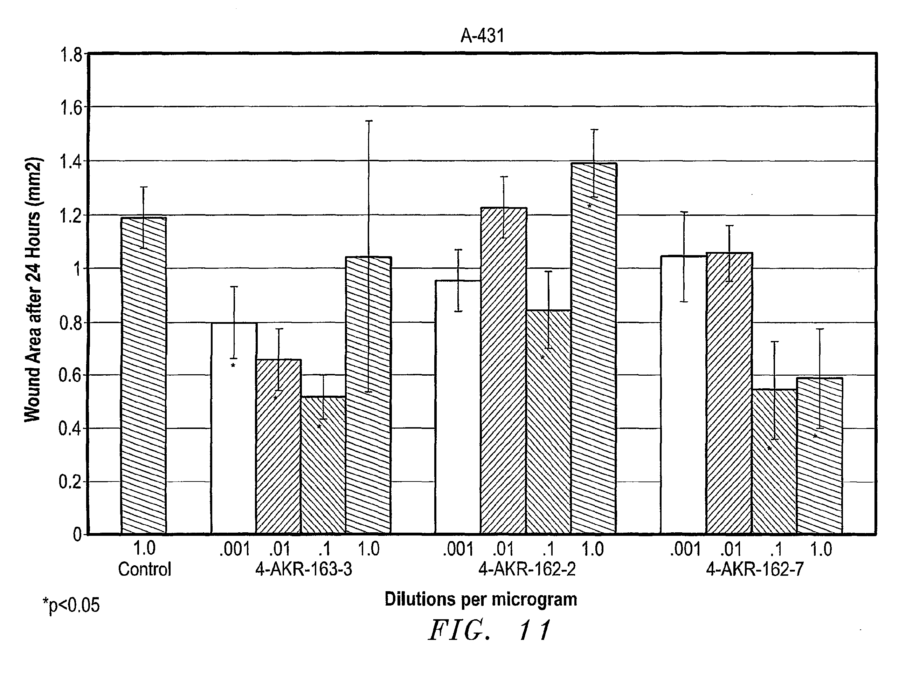

Fig. 11 is a chart of the wound area after 24 hours (mm²) vs. dilutions per microgram for the A-431 cell cultures of Example 14.
Fig. 17 is a cross-sectional view of a wound dressing along line A-A in Fig. 17A.
Figure 18 is a graph illustrating re-epithelialization of keratin treated wounds in a porcine model compared to an occlusive dressing control (Example 16).
Fig. 19. is a graph illustrating neovascularization of keratin treated wounds in a porcine model compared to an occlusive dressing control (Examples 16).

### Detailed Description of the Invention

The present invention is defined in the claims and provides tissue defect dressings comprising a keratinaceous salve, and provides methods for treating tissue defects using these dressings.

As used herein, the phrase "tissue defect," includes, but is not necessarily limited to wounds, ulcers, bums, natural defects, such as birth defects, and any other defects of bodily tissue, including skin, bone, cartilage, gastrointestinal organs, and other internal organs. The term "wound" refers to damage to any tissue in a living organism. A wound may be in a soft tissue, such as the spleen, or in a hard tissue, such as bone. A wound may have been caused by any agent, including traumatic injury, disease, infection, surgical intervention, or natural causes.

Preferred tissue defects for treatment with the dressing described herein are skin defects. Examples of treatable skin defects include, but are not necessarily limited to dry skin, eczema, dermatitis, psoriasis, acne, hives, athlete's foot, impetigo, cellulitis, abrasions, incisions, punctures, skin cancers, cysts, rashes, keloid scars, hypertrophic scars, birth defects, and cosmetic defects, such as winkles.

Certain proteins, such as keratin, are inherently biocompatible. Elastomeric films and hydrogels comprising keratin networks have been made and have proven useful and effective to treat tissue defects. Foams comprising keratin networks also have been made. The tissue defect dressing of the present application comprises a biocompatible "proteinaceous salve" which is applied directly to the tissue defect. The "proteinaceous salve" comprises a proteinaceous network, preferably a keratinaceous network, which is integral with a suitable carrier. The salve may take a variety of forms, including but not necessarily limited to a film, a hydrogel, a foam, and the like. The proteinaceous network comprises "proteinaceous molecules," selected from the group consisting of peptides and protein molecules, comprising intermolecular associations consisting essentially of other than disulfide bonds. "Consisting essentially of other than disulfide bonds" is not intended to exclude the presence of disulfide bonds altogether, but to indicate that the type of associations crosslinking the protein/peptide molecules are primarily other than disulfide bonds.

It is possible to apply the proteinaceous salve in bulk form, i.e., by applying or spreading the proteinaceous salve, alone, onto the wound. However, the tissue defect dressings preferably are "cohesive," defined as comprising a substantially solid but elastic and pliable, self-supporting structure. It is possible to achieve a "cohesive" structure by manipulating the type and quantity of interprotein/interpeptide bonds in the proteinaceous network to increase the strength of the proteinaceous network, itself. In a preferred embodiment, a cohesive tissue defect dressing is achieved by permeating the proteinaceous salve into and/or bonding the proteinaceous salve onto a suitable support.

### The support

In a preferred embodiment, the tissue defect dressing comprises a support. The support may be substantially any material adapted to provide structure so that the dressing can be handled easily and which also serves as a semi-permeable barrier to moisture and keeps out pathogens. The precise structure of the support may vary depending upon the type of tissue defect. Many materials are used for this purpose and the choice of support can be made such that an optimal level of moisture retention and protection is afforded. See Acute & Chronic Wounds: Nursing Management, 2nd Edition, Ruth A. Bryant (editor), Mosby Inc., St. Louis (2000) .

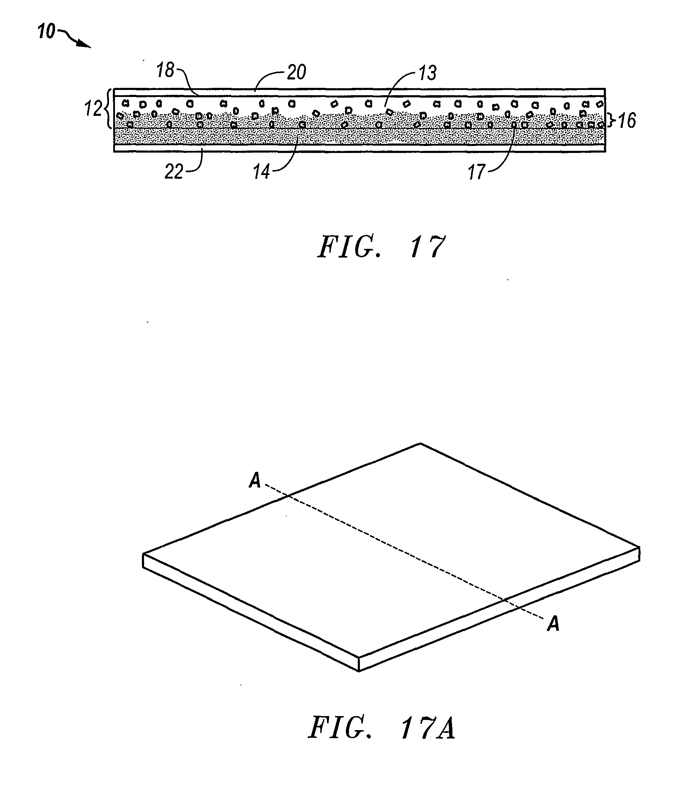

In a preferred embodiment (Fig. 17), the tissue defect dressing 10 comprises a proteinaceous salve 14 and a support 12 comprising a permeable layer 13, a bonding region 16, and a barrier layer 20. In a preferred embodiment, the support 12 is a substantially flat or planar sheet (Fig. 17A).

The permeable layer 13 preferably comprises a relatively porous material adapted to permit only a portion of the abutting proteinaceous salve 14 to permeate the permeable layer 13. Suitable materials for the permeable layer 13 include, but are not necessarily limited to permeable synthetic polymers, permeable natural fibers, and combinations thereof. A most preferred permeable layer 13 comprises cotton. The bonding region 16 may comprise any conventional material for bonding a gel material to a support material. For example, the bonding region 16 may comprise a biocompatible adhesive. In a preferred embodiment, the bonding region 16 comprises at least a portion of the proteinaceous salve 14 permeated into pores in the abutting surface 17 of the permeable layer 13. At an opposed surface 20 of the permeable layer 13 is a barrier material 20 adapted to provide a controlled moist environment, allowing moisture to permeate the tissue defect if it becomes too dry and also allowing moisture to escape from the tissue defect if it becomes too wet. Suitable barrier materials include, but are not necessarily limited to semipermeable adhesive films, perforated films, natural or synthetic cloth, nonwoven films and webs, and extruded films. The support used in the examples is a TELFA^{®} pad (Beiersdorf, Inc., Wilton, CT) from which the inner polymer lining is removed to expose the inner cotton core which is bonded to a keratinaceous hydrogel.

The tissue defect dressing 12 preferably is enclosed in a conventional casing and sterilized using conventional means. For example, the tissue defect dressing may be enclosed in a porous paper sheet which permits entry of a sterilizing gas, such as ethylene oxide, but is impervious to pathogenic organisms. Exposure to ethylene oxide for a period of about 360 minutes generally is satisfactory for sterilization. Alternately, the material may sterilized by exposure to ionizing radiation from a gamma radiation source. An exemplary paper sheet is 17.00 kg (37.5-pound) per ream porous, waterproof paper, e.g. TYVEK^{™} paper (available from DuPont, Inc. of Wilmington, Del.). The proteinaceous salve 14 preferably is protected by a conventional plastic sheet 22, which may be simply peeled off before application. An example of a suitable the plastic sheet is a 5 mil laminate, e.g. of polyethylene, aluminum foil and MYLAR^{™} as available from Technipaq Corporation of Chicago, Ill. Where a TELFA^{®} pad is used, the inner polymer lining may be reapplied to the surface of the proteinaceous salve, and the structure encased and sterilized. Various structures may be provided at one end of the pouch or casing to facilitate opening.

In order to treat a tissue defect, the surface of the proteinaceous salve is applied directly to the tissue defect. The tissue defect dressing is strong enough to allow for easy removal and to provide some cushioning for the wound bed, i.e., protecting the wound. The dressing, particularly the hydrogel, contains a large quantity of moisture that will maintain the wound in a moist condition. The dressing also absorbs exudate from the wound as well as evaporates moisture from its top surface. The dressing will remain in place after application but can be easily removed when required.

### Delivery of Bioactive Components

The hydrogels and heterogeneous films are shown herein to serve as a delivery vehicle for bioactive components present in the keratin, itself. The hydrogels and films also are useful as a delivery vehicle for other therapeutic and bioactive components, including but not necessarily limited to growth factors, cytokines, chemotactic agents, pharmaceuticals, proteins, cells, and extracellular components. In order to prepare hydrogels or films containing such bioactive components, the bioactive components are simply added to the solution used to form the hydrogel or film.

### Keratin

A large variety of proteins and/or peptides may be used to form the networks in the proteinaceous salve. The proteins and/or peptides preferably have a molecular weight of at least 10 kDa, more preferably at least 40 kDa, and most preferably 60 kDa or more. The proteins are biocompatible, with the result that they inherently provide control over biological compatibility. Examples of suitable naturally occurring proteins include, but are not necessarily limited to proteins derived from keratin, collagen, and elastin. The proteins may be derived from natural, synthetic, or recombinant sources. A preferred source of keratin proteins is hair or fur. The hair may be animal, or human. Preferred proteins are relatively high in cysteine content. Most preferred proteins are keratins, preferably water soluble keratins, even more preferably water soluble α-keratins, most preferably water soluble high molecular weight keratins (HMWK's).

Keratins are loosely defined as the hardened and insolubilized proteins found in the epidermal cells of vertebrates. Human hair is composed almost entirely of keratins. Human hair has a cuticle, which is a tough tubular outer layer made up of flattened cells arranged in a scaly, overlapping profile. The inner bulk of the hair is called the cortex and is constructed from elongated cells that are densely packed with fibrous keratins. The fibrous keratins are arranged in bundles referred to as microfibrils and possess an α-helical tertiary structure. The microfibrils are bound together with an amorphous keratin matrix.

The amorphous keratin matrix and the microfibrils vary in function and composition. The matrix is the "glue" that holds the microfibrils together. This matrix "glue" is high in sulfur content, and is comprised of low molecular weight keratins (LMWK) which typically have an average molecular weight of from 10 to 15 kDa. The microfibrils are comprised of α-keratins, including high molecular weight keratins (HMWK), having a relatively lower sulfur content, but having a higher average molecular weight of typically from 50 to 85 kDa. HMWK's and LMWK's vary in chemical properties, such as reactivity and solubility.

Keratins are afforded their structural integrity, in large part, by the presence of disulfide crosslinks which form a three dimensional network of polyprotein chains. This network structure renders keratins insoluble. Keratins can, however, be made water soluble by destroying this three dimensional structure via disulfide bond scission. Disulfide bond scission can be performed either oxidatively, reductively, or using some combination of both types of bond scission. Oxidative bond scission with hydrogen peroxide, for example, results in the formation of sulfonic acid residues produced from cystine. The material produced using hydrogen peroxide for disulfide bond scission is highly ionic and has excellent water solubility. Reductive bond scission with mercaptoethanol, for example, results in the formation of cysteine residues produced from cystine. The material produced using this reductive technique is highly reactive and will readily re-crosslink.

### Hydrogels

In a preferred embodiment, the proteinaceous salve is a hydrogel comprising a proteinaceous network derived from keratin. Hydrogels are networks of hydrophilic macromolecules that swell in water, but do not dissolve. Hydrogels are used in a variety of medical applications. The network of hydrophilic macromolecules can comprise polymer entanglements (virtual crosslinks), pseudo crosslinks, and/or covalent crosslinks. The point at which the molecular weight of a given macromolecule is sufficient for virtual crosslink formation is known as the critical entanglement molecular weight (MW_{c}). At the MW_{c}, a virtual network is formed, the molecular weight of the material is essentially infinite, and the viscosity increases exponentially as the material reaches its gel point. The macromolecules form pseudo crosslinks when a coordinating molecule attaches itself to two adjacent macromolecules, thus forming a "bond" between them. These types of "bonds" typically are electrostatic in nature, the most common being ionic and hydrogen bonds. Covalent crosslinks typically form the most stable networks.

Regardless of whether the network crosslinks are virtual, pseudo, and/or covalent, the hydrogel resists dissolution because of the nominally infinite molecular weight resulting from the crosslinks. The type of crosslinks found in the network controls the mechanical characteristics of the resulting hydrogel, as well as the in vivo degradation rate as the network is acted upon at the cellular and molecular level.

Hydrogels have the advantage that they can have a relatively high solids content while retaining a high level of biocompatibility. The proteinaceous networks preferably comprise water soluble proteins, preferably water soluble keratins, crosslinked in a manner that affords broad control over mechanical, chemical, and biological properties. The crosslinking preferably consists essentially of hydrolyzable and non-hydrolyzable crosslinks to provide control over biodegradability.

In order to make a hydrogel using human hair, sufficiently high molecular weight α-keratins from within the microfibrils are selectively extracted and isolated, preferably as described above. Although the exact MW_{c} for HMWK's is not known, the molecular weight of HMWK's is sufficiently high for virtual crosslink formation and creation of a three-dimensional keratin network. The keratins in the network are hydrophilic in nature. As a result, the three-dimensional keratin network may be swelled with water to produce a hydrogel. Gelation and swelling can be enhanced by the presence of ions. The keratin network is able to sequester the ions, which form pseudo crosslinks and make the network even more hydrophilic. These hydrogels are preferred for some applications.

Unfortunately, hydrogel networks formed solely of critical entanglements and/or pseudo crosslinks may not be sufficiently stable for many uses since the proteins in such hydrogel networks are not covalently linked. In order to produce truly stable hydrogels that exhibit little to no "creep," covalent crosslinks must be introduced into the hydrogel.

The present application describes suitable covalent crosslinks for forming more stable proteinaceous, preferably keratinaceous hydrogels, and provides methods for forming proteinaceous networks comprising such covalent bonding. The hydrogels comprising covalent crosslinks may be engineered to produce a predetermined rate of biodegradation. The rate of biodegradation is controlled by (a) providing hydrolyzable and/or non-hydrolyzable covalent bonding between the proteins in the hydrogel; and (b) manipulating the accessibility of the covalent crosslinks to water molecules.

Covalent bonds that are susceptible to hydrolytic cleavage, such as ester or ether bonds, can be used to impart a relatively rapid biodegradation rate. Conversely, hydrolytically stable bonds such as carbon-carbon, carbon-nitrogen, and carbon-sulfur bonds can be used to extend *in vivo* biostability. The accessibility of the crosslink to water molecules is manipulated by the introduction of steric hindrance, preferably by flanking susceptible bonds with hydrophobic groups to impart hydrolytic stability.

### Disulfide bond scission and keratin extraction

The following methods are preferred for processing keratins to produce hydrogels. Although the hydrogels may be made using other proteins, the following description refers to keratins for the sake of simplicity.

The keratins may be processed and/or isolated in a number of ways. Preferably, the processing is sufficient to render the resulting proteins water soluble. Suitable processing techniques include known oxidation techniques, reductive techniques, and/or combinations thereof, as long as the processing renders the proteins water soluble without significant hydrolysis of peptide bonds. At least in the case of keratins, preferred proteins are HMWK's which are processed and isolated by a two step reduction process.

A number of reductive chemistries are known for disulfide bond scission in keratins: See Wardell, J. L., "Preparation of Thiols" in The Chemistry of the. Thiol Group, Patai, S. (Editor), pp. 163-353, John Wiley & Sons, New York, NY (1974); . HMWK's may be extracted from hair using at least two reductive extractions, as described in Crewther, W. G., Fraser, R. D. B., Lennox, F. G., and Lindley, H., "The Chemistry of Keratins" in Advances in Protein Chemistry, Anfinsen, C. B., Jr., Anson, M. L., Edsall, J. T., and Richards, F. M. (Editors), Academic Press, New York, pp. 191-346 (1965).

In a preferred embodiment, a first reductive extraction is performed by treating the hair with a first reducing agent under first conditions effective to selectively extract matrix keratins, producing a first solution comprising soluble reduced matrix keratins (LMWK's) and remaining hair solids (HMWK's). Although it may be possible to subject the LMWK's to the techniques described herein to produce hydrogels, preferred proteins for use in the techniques herein are HMWK's, which preferably are isolated during a second extraction. The remaining hair solids and the first solution are separated, and the remaining hair solids are exposed to a second extraction solution under second conditions effective to solubilize α-keratins, producing a second solution comprising soluble reduced α-keratins (HMWK's) and solid cuticle.

Suitable reducing agents include, but are not necessarily limited to thioglycolic acid and salts thereof, mercaptoethanol, dithiothreitol, thioglycerol, thiolactic acid, glutathione, cysteine, sodium sulfide, and sodium hydrosulfide. Preferred reducing agents are thioglycolic acid and mercaptoethanol, most preferably thioglycolic acid.

In order to selectively reduce and extract the desired proteins, the hair (or other protein source) is suspended in a reducing agent at a concentration of from 0.1M to 10M, preferably 1.0M. Gentle swelling of hair fibers is achieved at a pH of 9 or more, preferably at a pH of from 9 to 10.5. Hence, the initial reduction takes place at a temperature of from 20 to 100 °C, preferably at 25°C. The time period required to accomplish the first reduction is from 4 24 hours, most preferably 12 hours. The reaction occurs under an inert atmosphere, preferably nitrogen. The liquid fraction is separated from remaining solids using known means, including but not necessarily limited to filtration, cannulation, and/or centrifugation, preferably under inert atmosphere. A preferred method of separation is filtration.

A second extraction is performed using a suitable swelling agent, preferably urea, bases such as ammonium hydroxide, sodium hydroxide, or potassium hydroxide. A most preferred swelling agent for this second extraction is concentrated urea. The second extraction effectively removes the fibrous α-keratins from inside the cuticle. The second extraction occurs at from 1M to 10M urea, preferably 7M urea, for a period of at least 1 hour, preferably from 1 to 72 hours, most preferably 24 hours. The second extraction occurs at room temperature, but may take place at temperatures of from 20°C to 100 °C, preferably 25 °C. The liquid fraction is separated from the empty, intact cuticle, using known means. Suitable means include but are not necessarily limited to filtration, cannulation and/or centrifugation, preferably under inert atmosphere. A preferred method of separation is filtration.

Once the cuticle is removed, the water soluble keratin proteins may be retained in solution for further use, or they may be isolated from the solution by addition to a water-miscible non-solvent, or by spray drying. Water-miscible non-solvents include, but are not necessarily limited to ethanol, methanol, isopropyl alcohol, tetrahydrofuran, acetone, dioxane, and the like, again under inert atmosphere. A preferred non-solvent is ethanol. The precipitate is separated from the non-solvent using known means, preferably by filtration and rinsing using additional aliquots of the non-solvent. The precipitated proteins are dried using known techniques, preferably overnight under vacuum at room temperature. The extracted water soluble keratin proteins (herein sometimes collectively referred to as "water soluble proteins") comprise thiols or thiol groups.

Thiols possess reactivities similar to alcohols, and can be used to perform a multitude of known organic chemical reactions, such as those described in McMurry, J., Organic Chemistry, Brooks/Cole Publishing Co., Monterey, CA (1984); Scudder, P. H., Electron Flow in Organic Chemistry, John Wiley & Sons, New York, NY (1992); Stowell, J. C., Intermediate Organic Chemistry, John Wiley & Sons, New York, NY (1994). The rate of reduction is affected by reagent concentration(s), reaction temperature(s), and exposure time(s).

### Formation of Covalent Crosslinks

Thiols and other chemical moieties contained in amino acid residues have utility as labile sites for covalently crosslinking the water soluble proteins. Preferred reactions are reactions that form other than disulfide bonds.

In a preferred embodiment, crosslinking agents are used to produce covalent bonding. Preferred crosslinking agents produce biocompatible byproducts, preferably hydrogen, water, carbon dioxide, and/or any other biocompatible byproduct that is readily metabolized or excreted, removed from the network, or at least is not toxic to the human body.

In order to prepare the networks, the desired crosslinking agent(s) are determined. Crosslinking agents other than glutaraldehyde having two or more of the same functional groups, or two or more different functional groups are suitable. Preferable crosslinking agents have two or more of the same functional group, as described below.

In a preferred embodiment, the HMWKs are provided as a dry powdered precursor. If a crosslinking agent is used, the crosslinking agent is provided in an aqueous solution which is added to the powdered HMWKs. Upon mixing, a viscous hydrogel results. The aqueous solution of crosslinker is prepared such that at least 5 wt.%, preferably 10 wt.%, relative to the keratin, of the multifunctional crosslinking agent is added, forming a hydrogel. Depending upon the crosslinking agent, a catalyst or promoter may be added. The hydrogel may also be formed first by adding the water to the HMWK powder, followed by adding the crosslinking agent and a catalyst.

### Crosslinking reactions

Crosslinking of the water soluble proteins and network formation occurs, generally, when a non-reactant which is at least difunctional, or has at least two reactive groups, is used to crosslink between reactive pendant groups on two different water soluble keratin proteins. The non-protein reactant creates a bridge between the water soluble proteins, thereby producing a three-dimensional network.

Proteins comprise amino acids, which generally have the formula: 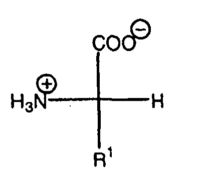 Table 1 summarizes the amino acid residues found in human hair, for example, and shows the "R¹" groups associated with each residue.

| Table 1. Ranked average amounts of amino acids in human hair | | | | | |
|---|---|---|---|---|---|
| Amino Acid | R¹ Group | Nature | pKa | Isoelectric Point (pH) | Percent Composition in Hair |
| Cysteine | H-S-CH₂- | Nonpolar | 8.4 | 5.02 | 17.3 |
| Glutamic Acid | 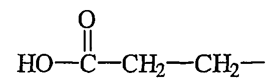 | Polar | 4.5 | 3.22 | 13.9 |
| Arginine | 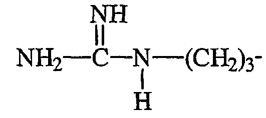 | Polar | 12.5 | 11.15 | 9.85 |
| Serine | HO-CH₂- | Polar | None | 5.68 | 9 |
| Threonine | 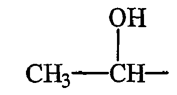 | Polar | None | 5.64 | 7.75 |
| Leucine | 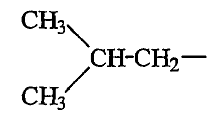 | Hydrophobic | None | 5.98 | 7.35 |
| Proline | 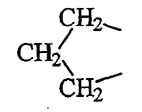 | Hydrophobic | None | 6.3 | 6.95 |
| Aspartic Acid | 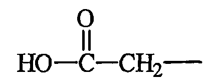 | Polar | 4.5 | 2.77 | 5.8 |
| Valine | 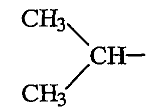 | Hydrophobic | None | 5.96 | 5.7 |
| Isoleucine | 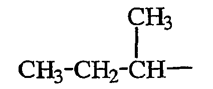 | Hydrophobic | None | 5.94 | 4.75 |
| Glycine | H- | Nonpolar | None | 5.65 | 4.15 |
| Phenylalanine | 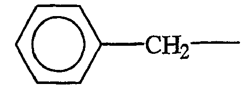 | Hydrophobic | None | 5.48 | 3 |
| Alanine | CH₃- | Hydrophobic | None | 6 | 2.8 |
| Tyrosine | 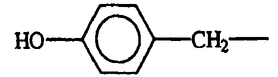 | Hydrophobic | None | 5.66 | 2.6 |
| Lysine | NH₂-(CH₂)₄- | Polar | 10.4 | 9.59 | 2.5 |
| Histidine | 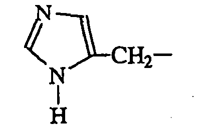 | Aromatic | 6.2 | 7.47 | 0.9 |
| Methionine | CH₃-S-CH₂-CH₂- | Hydrophobic | None | 5.74 | 0.85 |
| Tryptophan | 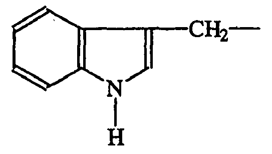 | Hydrophobic | None | 5.89 | 0.85 |

The most abundant amino acid in human hair is cysteine, which is found in the form of disulfide-bridged cystine groups. As discussed above, this group can be converted to other sulfur containing moieties, most notably thiol. Thiols theoretically can be reacted with reactive ends of a crosslinking agent using a number of chemical techniques, such as those described in S. Patai (Ed.), the Chemistry of the Thiol Group, Parts 1 and 2, John Wiley & Sons, New York, NY (1974) . Other reaction scenarios, such as those directed toward polymer synthesis, also are useful to utilize thiols to form an assortment of desirable crosslinks, including those described in Rempp, P. and Merrill, E. W., Polymer Synthesis, Huethig & Wepf Verlag Basel, Heidelberg, Germany (1986); Young, R. J. and Lovell, P. A., Introduction to Polymers, Chapman & Hall, London (1991); Odian, G., Principles of Polymerization, John Wiley & Sons, New York, NY (1991).

In addition to cysteine, the following amino acids have pendant groups comprising nitrogen or oxygen which may be useful as reactive pendant groups; arginine, serine, glutamic acid, threonine, aspartic acid, lysine, asparagine, glutamine, tyrosine, tryptophan, and histidine. Where the protein is α-keratin, preferred amino acid residues comprising reactive pendant groups for crosslinking are cysteine, arginine, serine, and glutamic acid, most preferably cysteine and arginine.

Crosslinking agents comprise at least two reactive groups. Preferred reactive groups are selected from the group consisting of epoxide groups, isocyanate groups, and carboxyl groups. Most preferred crosslinking agents are diepoxides, diisocyanates, and dicarboxylates, including anhydrides and hydrolyzed diacids thereof.

Other suitable crosslinking agents include, but are not necessarily limited to polyethylene glycols (PEGs), multi-arm PEGs, and polyethylene glycol derivatives. Suitable polyethylene glycol derivatives include, but are not necessarily limited to nucleophilic PEGs; electrophilically active PEGs, heterofunctional PEGs, PEG-biotins, vinyl derivatives, and PEG phospholipids. Exemplary crosslinking agents include, but are not necessarily limited to: mPEG-acetaldehyde diethyl acetal; mPEG-acrylate; mPEG2-aldehyde; mPEG-amine; ω-amino-α-carboxyl PEG; mPEG-benzotriazole carbonate, PEG-biotin, t-Boc-protected amine; mPEG-double esters; fluorescein-PEG-NHS; FMOC protected amine; mPEG forked maleimide; mPEG maleimide; multi-arm PEG; NHS-PEG- maleimide; NHS-PEG-vinlsulfone; mPEG2-N-hydroxysuccinimide; PEG-phospholipid; mPEG propionaldehyde; mPEG succinimidyl butanoate, and mPEG succinimidyl proprionate, available from Shearwater Corporation, Huntsville, Alabama.

For convenience, the crosslinking agents described herein sometimes are referred to as "di-" functional. However, unless a crosslinking agent is expressly claimed or expressly stated to be di-functional only, it is to be understood that the crosslinking agents described herein may also be multi-functional, e.g., di-, tri, tetra-, etc. The non-functional portion of the molecule (R⁵, below) generally forms the remainder of the , "bridge" crosslinking the proteins. R⁵ is biocompatible and typically is an organic moiety. Suitable organic moieties include, but are not necessarily limited to alkoxy groups, alkylene groups, and alkenyl groups having from 1 to 50 carbon atoms. The alkoxy groups, alkylene groups, or alkenyl groups may be present alone, or in combination with cyclic alkyl groups or aromatic groups.

Without limiting the claims to a particular theory or mechanism of action, unless expressly claimed, the following are crosslinking chemistries involved in producing the crosslinked water soluble protein networks:

### Addition to Amine Groups

A preferred, covalently crosslinked hydrogel is made by addition reactions between reactive amine groups and oxirane compounds. Such addition reactions occur readily without the aid of a catalyst. The crosslinking reaction with arginine is as follows: 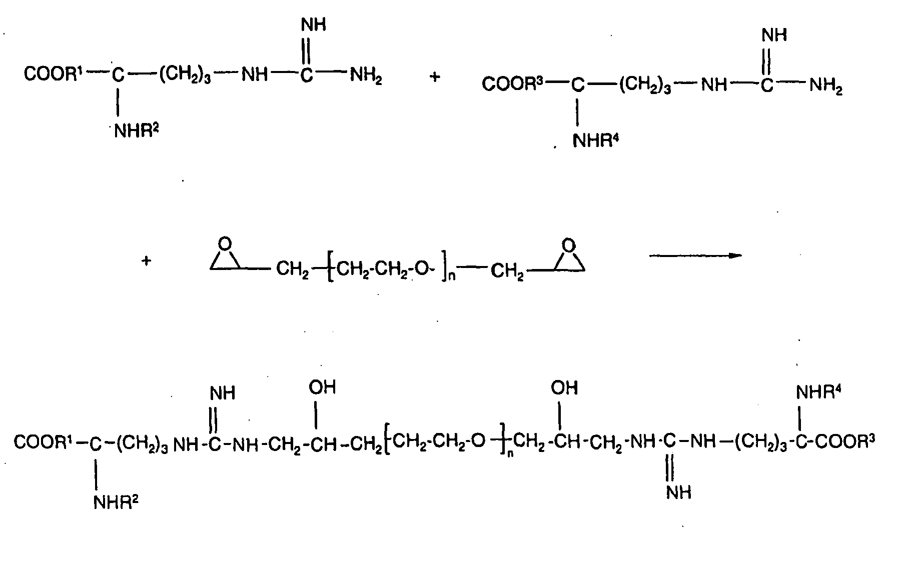 wherein R¹ and R² represent the remainder of one water soluble protein molecule and R³ and R⁴ represent the remainder of a separate water soluble protein molecule, preferably different water soluble α-keratin molecules.

In order to perform this reaction, the water soluble keratins are exposed to a solution containing an oxirane-containing aliphatic or aromatic compound, typically at a concentration of up to 20 weight percent relative to keratin, preferably between 5 and 10 weight percent relative to keratin; at a pH between 4 and 9, preferably 7; at a temperature of from 0 to 100°C, preferably 37°C, preferably for a time period of from 0 to 72 hours, most preferably 24 hours. This process results in the formation of a hydrogel within 1 to 2 minutes and the crosslinking reaction occurs while the keratins are in the gelled state.

A preferred oxirane compound is a diepoxide having the following general structure: 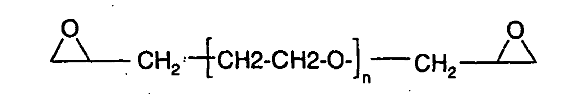 wherein n is from 1 to 50. Preferred epoxides include DER^{™} 332 and DER™ 736, available from the Dow Chemical Company.

A similar reaction occurs when a diepoxide reacts with cysteine residues: 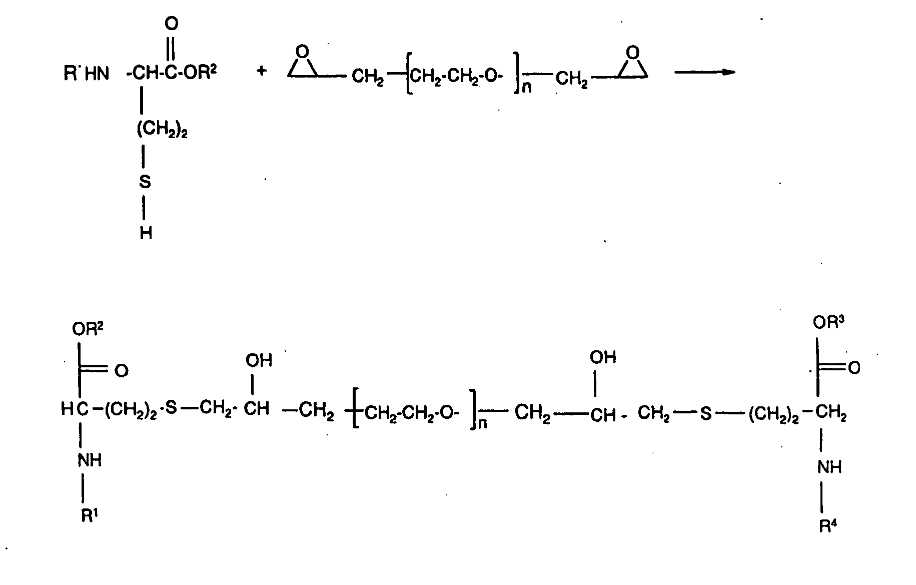 wherein n is from 1 to 50, R¹ and R² are the remainder of a first water soluble protein molecule and R³ and R⁴ are the remainder of a second water soluble protein molecule.

In order to perform these reactions, the crosslinking agent is preferably dissolved in an anhydrous solvent, such as methanol, ethanol, isopropyl alcohol, dimethylsulfoxide, acetone, or tetrahydrofuran. The concentration of this solution is such that the appropriate amount of crosslinking agent is added as the hydrogel is formed. For example, to form a hydrogel with 10 wt. % crosslinking agent and 5 wt. % HMWK solids, 1 gram of a solution containing 0.1 grams of crosslinking agent dissolved in the anhydrous solvent is added to 1.0 gram of HAWK powder dissolved in 18 gm of water. The solution and powder are thoroughly mixed and a hydrogel spontaneously forms. This hydrogel is allowed to react under the time and temperature conditions described previously to effect crosslinking. Crosslinking reactions can be accelerated by incubating this gel in a closed atmosphere at a temperature of from 25 °C to 80 °C, preferably 37 °C.

Persons of ordinary skill in the art will recognize that many of the crosslinking agents described herein will react with a variety of amino acid residues having pendant groups comprising a reactive nitrogen atom, sulfur atom, or oxygen atom. Hence, one end of a diepoxide may react with a cysteine residue while the other end of the diepoxide reacts with an arginine residue, as follows:

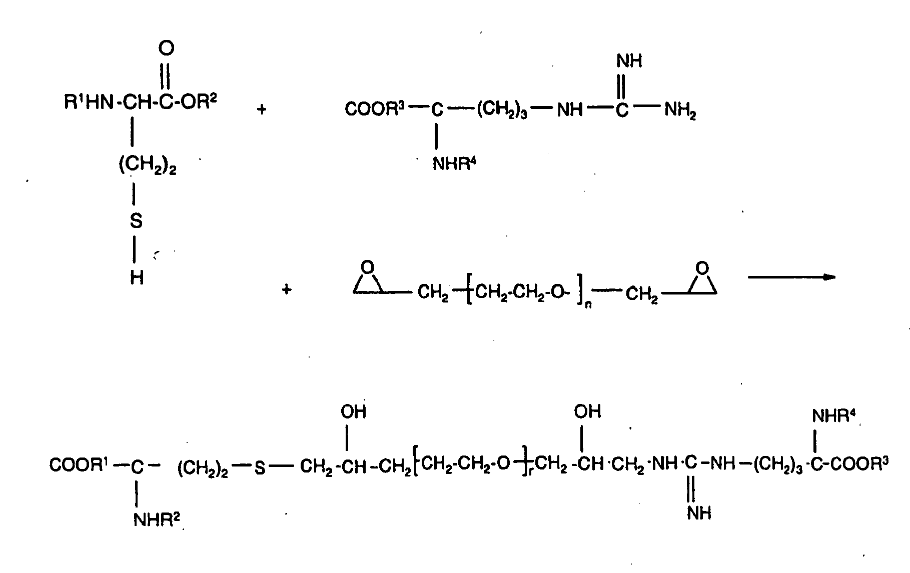

The identity of amino acid residues linked by the crosslinking agent is not as important as the requirement that a sufficient quantity of crosslinking between water soluble proteins occurs to produce a network and preferably a hydrogel having desired properties.

### HETEROGENOUS NETWORKS OR FILMS

In an alternative tissue defect dressing, the proteinaceous salve comprises a heterogeneous crosslinked proteinaceous network. As used herein, the term "heterogeneous" refers to a proteinaceous network or film, preferably comprising protein molecules having a relatively high molecular weight of from 50 to 85 kDa, or derivatives therefrom. The protein molecules are interlinked by a non-proteinaceous crosslinking material.

As with hydrogels, a variety of proteins can be used to form heterogeneous network structures, preferably elastomeric films. Examples of suitable naturally occurring proteins include, but are not necessarily limited to keratin, collagen, and elastin. The proteins may be natural, synthetic, or recombinant. Preferred proteins are relatively high in cysteine content. Most preferred proteins are keratin proteins, even more preferably α-keratin proteins, also sometimes called high molecular weight keratins (HMWK's).

### Disulfide bond scission and keratin extraction

The proteins, preferably α-keratins, may be processed and/or isolated in any manner that renders them sufficiently soluble in the reaction media for crosslinking reaction(s) to occur. A number of the reactions described below call for an anhydrous solvent. Persons of ordinary skill in the art will recognize that anhydrous solvents include a large number of solvents, including, but not necessarily limited to 1,2,-dimethoxyethane, dimethylformamide, dimethylsulfoxide (DMSO), N-methyl pyrrolidone, and others. Generally, the reactions require the presence of at least some water.

### Oxidation/Reduction of Cystine Residues

In a preferred embodiment, which uses keratins as a source material (e.g. human hair), the hair is oxidized by a suitable oxidizing agent. Suitable oxidizing agents include, but are not necessarily limited to hydrogen peroxide, peracetic acid, percarbonates, persulfates, chlorine dioxide, sodium and calcium peroxides, perborates, and hypochlorite. The oxidants are used at a concentration of up to 35%, preferably at from 0.1% to 10%. The oxidation preferably occurs at reflux temperatures.

In a preferred embodiment, the hair is treated with hydrogen peroxide (H₂O₂), at from 0.1% to 10%, most preferably 1%, in order to disrupt the cuticle and swell the keratin source material. This process also converts some fraction of the cystine residues into sulfonic acid groups. The amount of oxidation may be controlled by varying the time of oxidation, preferably from 0 hours to 4 hours, while retaining the other conditions of the oxidation reaction constant. These conditions include concentration and type of oxidant, temperature, and ratio of extracting media to keratin source material. After the reaction is complete, the oxidized hair is filtered and rinsed, preferably with deionized water. The filtrate is discarded and the hair allowed to dry.

Where other conditions of oxidation are maintained constant, the conversion rate of cystine to sulfonic acid residues is roughly proportional to the amount of time used for the oxidation. Residual cystines in the resulting oxidized keratin solids are converted to other sulfur-containing moieties using reductive techniques. Preferably, the disulfide-bridged cystine group is converted to a thiol group, which has utility of it's own, or can be modified using a variety of chemical techniques.

### Reaction with a Reducing Agent

If oxidized, the oxidized hair preferably is treated with a reducing agent. Treatment of oxidized keratin proteins with reducing agents facilitates the formation of cysteine from cystine, but tends to leave the previously oxidized groups unaltered. Suitable reducing agents include, but are not necessarily limited to thioglycolic acid and salts thereof, mercaptoethanol, dithiothreitol, thioglycerol, thiolactic acid, glutathione, cysteine, sodium sulfide, and sodium hydrosulfide. Preferred reducing agents are thioglycolic acid and mercaptoethanol, most preferably thioglycolic acid.

In order to treat the oxidized hair with the reducing agent, the previously oxidized hair is suspended in the reducing agent typically at a concentration of up to 10N, preferably from 0.1N and 1N; at a pH greater than 7, preferably equal to or greater than 9, most preferably 9; a temperature of from 25 to 80 °C, preferably 60 °C, preferably for a time period of from 1 to 72, most preferably 24 hours. The reaction occurs under an inert atmosphere, preferably nitrogen. The liquid fraction is separated from any remaining solids using known means, including but not necessarily limited to filtration, or cannulation and/or centrifugation, preferably under inert atmosphere. A preferred method of separation is filtration. Once the solids are removed, the soluble keratin proteins are isolated from the solution by addition of a water-miscible non-solvent, or by spray drying. Water-miscible non-solvents include, but are not necessarily limited to ethanol, methanol, isopropyl alcohol, tetrahydrofuran, acetone, dioxane, and the like, again under inert atmosphere. A preferred non-solvent is ethanol. The precipitate is separated from the non-solvent using known means, preferably by filtration and rinsing using additional aliquots of the non-solvent. The resulting keratin proteins are dried using known techniques, preferably overnight under vacuum at room temperature. This process results in the keratins having both sulfonic acid groups and thiol groups.

Thiols possess reactivities similar to alcohols, and can be used to perform a multitude of known organic chemical reactions, such as those described in McMurry, J., Organic Chemistry, Brooks/Cole Publishing Co., Monterey, CA (1984); Scudder, P. H., Electron Flow in Organic Chemistry, John Wiley & Sons, New York, NY (1992); Stowell, J. C., Intermediate Organic Chemistry, John Wiley & Sons, New York, NY (1994). The ratio of sulfonic acid to thiol is primarily controlled by the quantity of primary reactive sites remaining after oxidation. Of course, the rate of reduction will also be affected by reagent concentration(s), reaction temperature(s), and exposure time(s).

### Reductive/reductive extraction

Reductive chemistries also are known for disulfide bond scission in keratins: See Wardell, J. L., "Preparation of Thiols" in The Chemistry of the Thiol Group, Patai, S. (Editor), pp. 163-353, John Wiley & Sons, New York, NY (1974) . HMWK's may be extracted from hair using at least two reductive extractions, as described in Crewther, W. G., Fraser, R. D. B., Lennox, F. G., and Lindley, H., "The Chemistry of Keratins" in Advances in Protein Chemistry, Anfinsen, C. B., Jr., Anson, M. L., Edsall, J. T., and Richards, F. M. (Editors), Academic Press, New York, pp. 191-346 (1965).

Briefly, in a first reductive extraction, the hair is treated with a first reducing agent under first conditions effective to selectively extract matrix keratins, producing a first solution comprising soluble reduced matrix keratins and remaining hair solids. The remaining hair solids and the first solution are separated, and the remaining hair solids are exposed to a second extraction solution under second conditions effective to solubilize α-keratins, producing a second solution comprising soluble reduced α-keratins and solid cuticle. Once the second extraction is complete, the remaining solids essentially are the empty, intact cuticle.

The liquid fraction is separated from the solid cuticle using known means, including but not necessarily limited to filtration, or cannulation and/or centrifugation, preferably under inert atmosphere. A preferred method of separation is filtration. Once the solids are removed, the soluble keratin proteins are isolated from the solution by addition of a water-miscible non-solvent, or by spray drying. Water-miscible non-solvents include, but are not necessarily limited to ethanol, methanol, isopropyl alcohol, tetrahydrofuran, acetone, dioxane, and the like, again under inert atmosphere. A preferred non-solvent is ethanol. The precipitate is separated from the non-solvent using known means, preferably by filtration and rinsing using additional aliquots of the non-solvent. The resulting keratin proteins are dried using known techniques, preferably overnight under vacuum at room temperature. The dried keratin proteins are ground into a powder, sometimes referred to as "HMWK powder."

### Network formation

In a preferred embodiment, which uses HMWK proteins, the HMWK proteins are dissolved in an aqueous solvent. Preferably, 2 g of HMWK powder is mixed in water containing a suitable base, and the mixture is stirred and heated to a temperature effective to dissolve the keratin, typically not more than 60°C. The pH of the solution is maintained at 9 to 11 using a suitable base. Suitable bases include, but are not necessarily limited to ammonium hydroxide, sodium hydroxide, and potassium hydroxide, preferably ammonium hydroxide. At least 5 wt.%, preferably 10 wt.%, relative to the keratin, of a multifunctional crosslinking agent is added to the mixture, forming a network precursor solution. Depending upon the crosslinking agent, a catalyst or promoter may be added. The network precursor solution is distributed over an appropriate surface or mold, preferably to a thickness of from 1 to 10 mm, and cured by exposure to suitable energy, such as a heat lamp, an autoclave, a microwave, or a UV lamp. In a preferred embodiment, the solutions are placed under a heat lamp effective to produce a temperature of at least 60°C for from 30 to 300 minutes.

### Crosslinking reactions

Crosslinking of the proteins and network formation occurs, generally, when a non-protein reactant which is at least difunctional, or has at least two reactive groups, is used to crosslink between reactive pendant groups on two different keratin molecules. The non-protein reactant creates a bridge between keratin molecules, and thus produces a three-dimensional network. The chemistries described above for the preparation of hydrogels also are useful for the preparation of heterogeneous films. A preferred embodiment for the preparation of heterogeneous films involves the use of diepoxides or oxiranes, and is described above in relation to hydrogels under the title "Addition of Amine Groups."

### Network properties

As seen, a three dimensional keratin-based network can be formed using a variety of chemistries. Preferably, the "dissolution rate" of such a network is controllable by controlling the crosslink density of the film and the level and type of functionality, particularly the functionality adjacent to the crosslink site. For example, the use of a crosslinking agent having one of the following characteristics reduces the dissolution rate of the resulting network: a crosslinking agent which forms S-C bonds, as opposed to more hydrolyzable bonds, such as ester bonds; a crosslinking agent which introduces substantial steric hindrance at the crosslink site; a crosslinking agent which is hydrophobic. The "dissolution rate" of the resulting network or film is measured by determining how long the film resists hydrolysis upon exposure to an aqueous buffer having a pH of 7. A desirable "dissolution rate" will depend upon the application in which the film or hydrogel is to be used.

The invention will be better understood with reference to the following Examples:

### Example 1

800 mL of 0.8M thioglycolic acid (TGA) at pH 10.2, adjusted by addition of 52.58 grams of potassium hydroxide (KOH), was added to a 1 L glass reactor containing 40 grams of human hair obtained from a local barber shop. The hair had been washed with an aqueous solution of mild detergent, rinsed, and air dried prior to use. The mixture was stirred gently at room temperature (ca. 25°C) under positive nitrogen pressure for 18 hours.

The reaction mixture was filtered and the extract titrated to pH 7 by addition of hydrochloric acid (HCl). The neutralized solution was added dropwise to a 10-fold excess of absolute ethanol, while stirring, to promote the formation of a precipitate. The precipitate was filtered and dried under vacuum.

The remaining reduced hair was further processed by first rinsing it free of residual reductant using copious amounts of deionized water, upon which the hair swelled to nearly twice its initial volume. The rinsed hair was placed in a 2 L reactor to which was added 400 mL of 7M urea solution. The reaction was stirred at room temperature under positive nitrogen pressure for 24 hours.

100 mL of the reaction mixture was centrifuged and filtered. The pH of the filtered extract was titrated to pH 7 with the addition of HCl. The neutralized solution was added dropwise to 900 mL of absolute ethanol to affect precipitation. The solid keratins were isolated by filtration and hydrated with 18 MΩ water, upon which, a thick hydrogel formed.

### Example 2

800 mL of 1M mercaptoethanol at pH 10.2, adjusted by addition of ca. 24 grams of KOH, was added to a 1 L glass reactor containing 40 grams of human hair obtained from a local barber shop. The hair had been washed with an aqueous solution of mild detergent, rinsed, and air dried prior to use. The mixture was stirred gently at room temperature under positive nitrogen pressure for 18 hours. After extraction, the mixture was filtered and the liquid discarded.

The remaining reduced hair was further processed by first rinsing it free of residual reductant using copious amounts of deionized water, upon which, the hair swelled to nearly twice its initial volume. The rinsed hair was placed in a 2 L reactor to which was added 400 mL of 7M urea solution. The reaction was stirred at room temperature under positive nitrogen pressure for 24 hours.

The reaction mixture was centrifuged and filtered. The pH of the filtered extract was titrated to 7 with the addition of HCl. The neutralized solution was added dropwise to 4,500 mL of absolute ethanol to affect precipitation. The solid keratins were isolated by filtration and dried overnight under vacuum. The dried keratin powder was ground to a medium consistency with a mortar and pestle.

1.0 grams of a 0.01M sodium hydroxide (NaOH) solution was added to a vial containing 0.1 grams of the keratin powder from Example 2, upon which, the mixture formed a thick hydrogel. Another 2.0 grams of 0.01M sodium hydroxide solution was added (3.0 grams total) which reduced the viscosity of the hydrogel only slightly. This process was repeated with 0.001M and 0.0001M NaOH solutions with similar results.

0.5 grams of a 30% ammonium hydroxide (MH₄OH) solution was added to a vial containing 0.1 grams of the sample from Example 2, upon which, the mixture formed a thick hydrogel. Subsequent additions of 0.5, 1.0, and 0.5 grams of NH₄OH did not appear to reduce the viscosity of the gel. Addition of a final 0.5 grams of NH₄OH (3.0 grams total) reduced the viscosity only slightly.

### Example 3

800 mL of 1M mercaptoethanol at pH 10.2, adjusted by addition of ca. 25 grams of KOH, was added to a 1 L glass reactor containing 40 grams of human hair obtained from a local barber shop. The hair had been washed with an aqueous solution of mild detergent, rinsed, and air dried prior to use. The mixture was stirred gently at room temperature under positive nitrogen pressure for 18 hours.

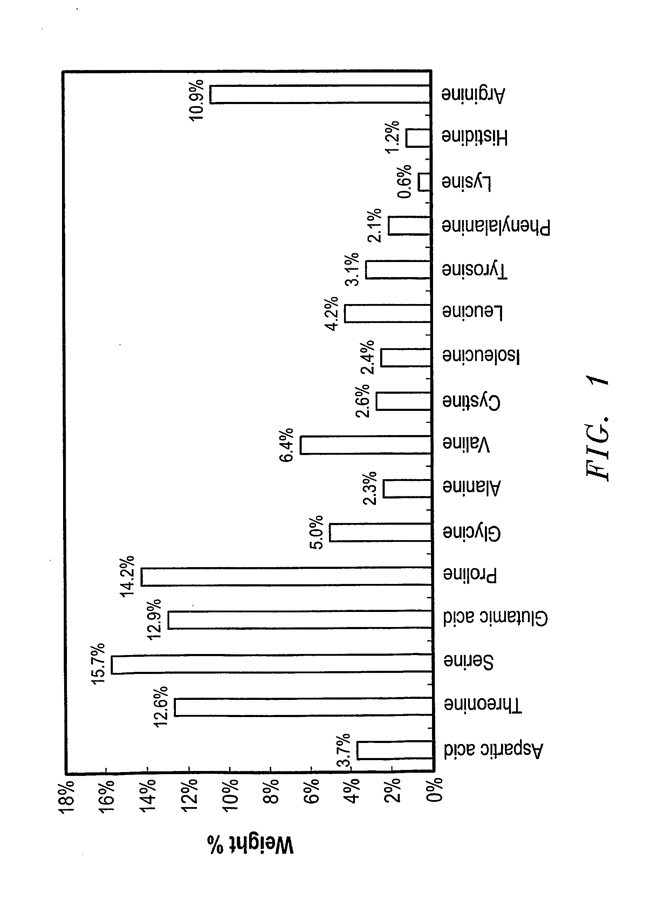

The reaction mixture was filtered and the extract titrated to pH 7 by addition of HCl. The neutralized solution was added dropwise to a 10-fold excess of absolute ethanol while stirring to promote the formation of a precipitate. The precipitate was filtered and dried under vacuum. Yield of keratin solids was 13.37 grams (33.4%). An amino acid analysis of this sample was conducted by acid hydrolysis and dissolution of the solid, followed by high pressure liquid chromatography (HPLC) of the solution. For cysteine residues, an analysis was performed on a separate sample by first oxidizing the solid keratins with performic acid, followed by acid digestion and HPLC analysis. The results of these analyses are summarized in the graph in Fig. 1.

The remaining reduced hair was further processed by first rinsing it of residual reductant using copious amounts of deionized water, upon which, the hair swelled to nearly twice its initial volume. The rinsed hair was placed in a 1 L reactor to which was added 400 mL of 7M urea solution. The reaction was stirred at room temperature under positive nitrogen pressure for 24 hours.

The reaction mixture was centrifuged and filtered. The pH of the filtered extract was titrated to 7 with the addition of HCl. The neutralized solution was added dropwise to 4,000 mL of absolute ethanol to affect precipitation. The solid keratins were isolated by filtration and dried overnight under vacuum. The dried keratin powder was ground to a medium consistency with a mortar and pestle. The yield of keratin solids was 1.66 grams (sample no. 4-AKR-112-2B; 4%).

Three sets of duplicate hydrogels containing 0.1 gram each of the keratin solids and 1.0, 2.0, and 3.0 grams of 30% NH₄OH solution were prepared in glass vials. The weight percent solids in each of these duplicate gels were 9.1, 4.8, and 3.2, respectively. The headspace of one of each of the duplicates was filled with pH 7.2 phosphate buffered saline and the vial capped and sealed with tape. These samples, as well as the set containing no buffer solution (also capped and sealed with tape) were placed in an incubator held at 37°C (body temperature) and monitored periodically by visual observation.

After 4 days in the incubator, the 2.0-gram gel in saline appear to begin disintegrating into the aqueous layer. There were no visible signs of any changes in the other hydrogels. After 16 days, all of the gels appeared intact, however, the buffer solutions displayed a slight brown discoloration. After 21 days, the gels were removed from the incubator. Other than a slight discoloration of the buffer solution, and a small amount of volume shrinkage in the buffered samples, no changes in any of the hydrogels appeared to have occurred.

### Example 4

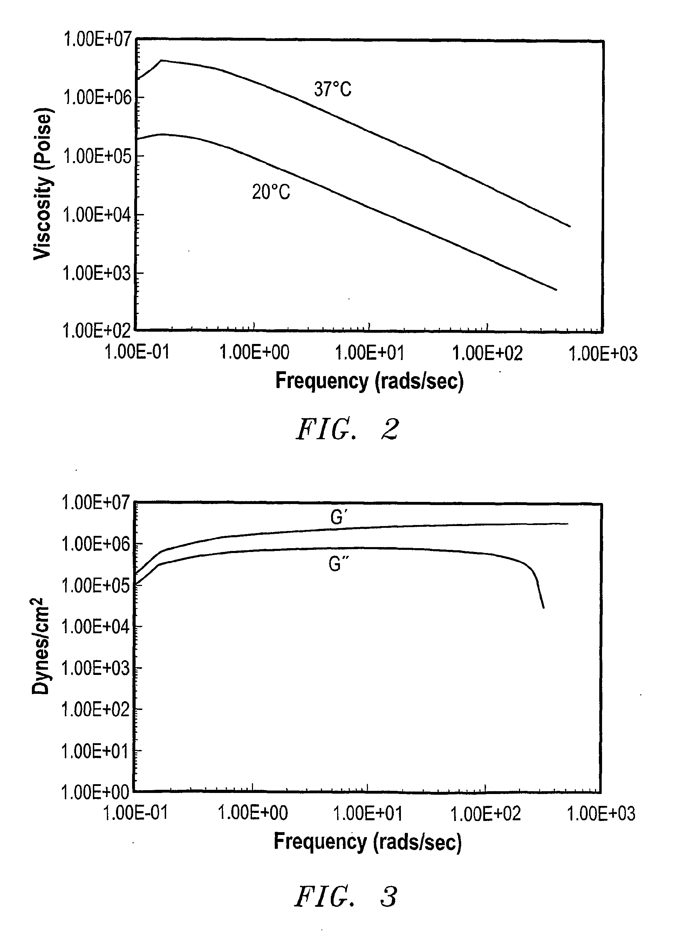

1.0 grams of a 30% ammonium hydroxide (NH₄OH) solution was added to a vial containing 0.1 grams of human hair from Example 3. The mixture formed a thick hydrogel. The viscosity of this gel was analyzed using a cone and plate rheometer. The results, shown in Figs. 2 and 3 suggest that the hydrogel is a classical non-Newtonian fluid.

In a "Newtonian fluid," the coefficient of viscosity at a given temperature and pressure is a constant for that fluid and independent of the rate of shear or velocity gradient. Non-Newtonian fluids consist of two or more phases present at the same time, and the coefficient of viscosity is not constant, but is a function of the rate at which the fluid is sheared as well as of the relative concentration of the phases. Non-Newtonian fluids frequently exhibit plastic flow, in which the flowing behavior of the material occurs after applied stress reaches a critical value or yield point (YP).

The fact that the hydrogel formed was a non-Newtonian fluid served to indicate that the bonding, such as pseudo-bonding or polymer entanglements had occurred within the gel.

### Example 5

3,500 mL of 1.0M thioglycolic acid (TGA) at pH 10.2, adjusted by addition of 312.5 grams of potassium hydroxide (KOH), was added to a 4 L glass reactor containing 175 grams of human hair obtained from a local barber shop. The hair had been washed with an aqueous solution of mild detergent, rinsed, and air dried prior to use. The mixture was stirred gently at room temperature (ca. 25°C) under positive nitrogen pressure for 18 hours.

The reaction mixture was sieved and the reduced hair was further processed by first rinsing it free of residual reductant using copious amounts of deionized water, upon which the hair swelled to nearly twice its initial volume. The rinsed hair was placed into two separate glass reactors to which was added 7M urea solution. These extractions were stirred at room temperature under positive nitrogen pressure for 24 hours.

The extracted hair was removed from solution by passing through a sieve. The liquid fraction was centrifuged and filter, then neutralized to pH 7 with the addition of HCl (3.7 mL). The liquid was added dropwise to a 10-fold excess of ethanol to effect precipitation of the HMWKs. The precipitate was isolated by filtration, washed with several aliquots of fresh ethanol, then dried overnight under vacuum. The resulting solid HMWKs were ground to a fine powder using a mortar and pestle.

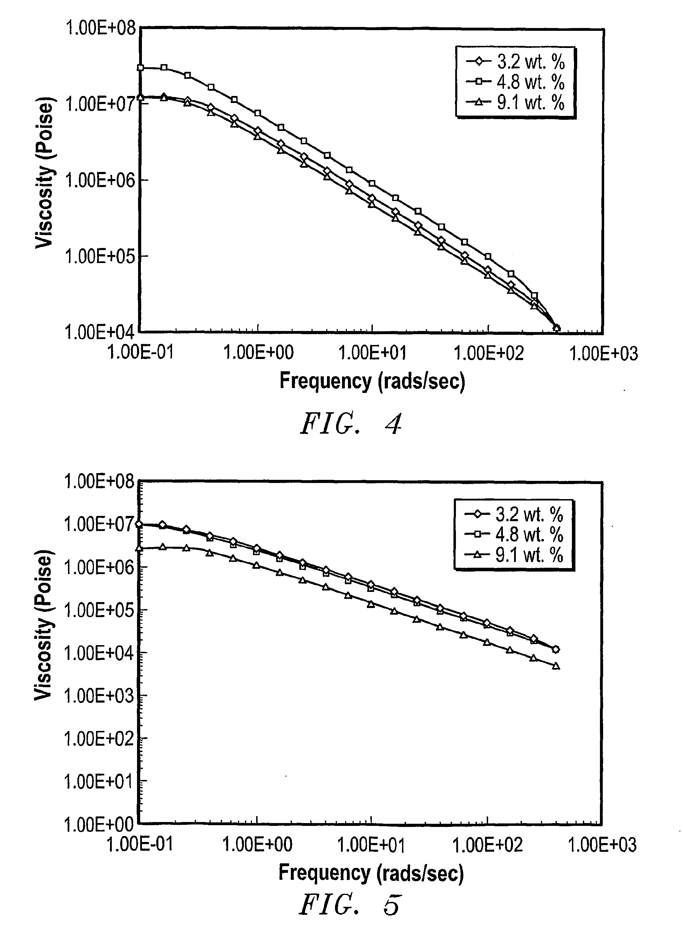

Nine-100 milligram samples of the powder were placed in glass vials. To each set of three vials was added 1.0, 2.0, and 3.0 grams of deionized water, respectively. The contents of each vial were mixed and allowed to gel. The resulting triplicate set of hydrogels contained 9.1, 4.8, and 3.2 weight % keratins, respectively. To one of each triplicate hydrogel was added enough pH 7.2 phosphate buffered saline solution to fill the headspace of the vial. These samples, as well as identical samples not containing buffer solution, were placed in an incubator held at a constant temperature of 37°C. The third sample of each triplicate was incubated at 37°C for 24 hours, then analyzed using a cone and plate rheometer. The rheometer was used to determine the shear dependant viscosity at 37°C of these "un-crosslinked" hydrogels. As used herein, "un-crosslinked" describes hydrogels that have not been reacted with a multifunctional crosslinker *per se;* such hydrogels will have virtual and pseudo crosslinks present. Fig. 4 shows the results for the 9.1, 4.8, and 3.2 wt. % hydrogels.

Similarly, nine-100 milligram samples of the powder were placed in glass vials. To triplicate samples was added 1.0, 2.0, and 3.0 grams of solutions containing 1.0, 0.5, and 0.33 wt. % of the crosslinker, DER 332 resin, respectively. This was done in order to deliver 0.01 grams (10 wt. % relative to keratin) of dissolved crosslinker to each vial of keratin powder.

To one of each triplicate hydrogel was added enough pH 7.2 phosphate buffered saline solution to fill the headspace of the vial. These samples, as well as identical samples not containing buffer solution, were placed in an incubator held at a constant temperature of 37°C. The third sample of each triplicate was incubated at 37°C for 24 hours, then analyzed using a cone and plate rheometer. The rheometer was used to determine the shear dependant viscosity at 37°C of these crosslinked hydrogels. Fig. 5 show the results for the 9.1, 4.8, and 3.2 wt. % hydrogels containing DER 332 resin crosslinks.

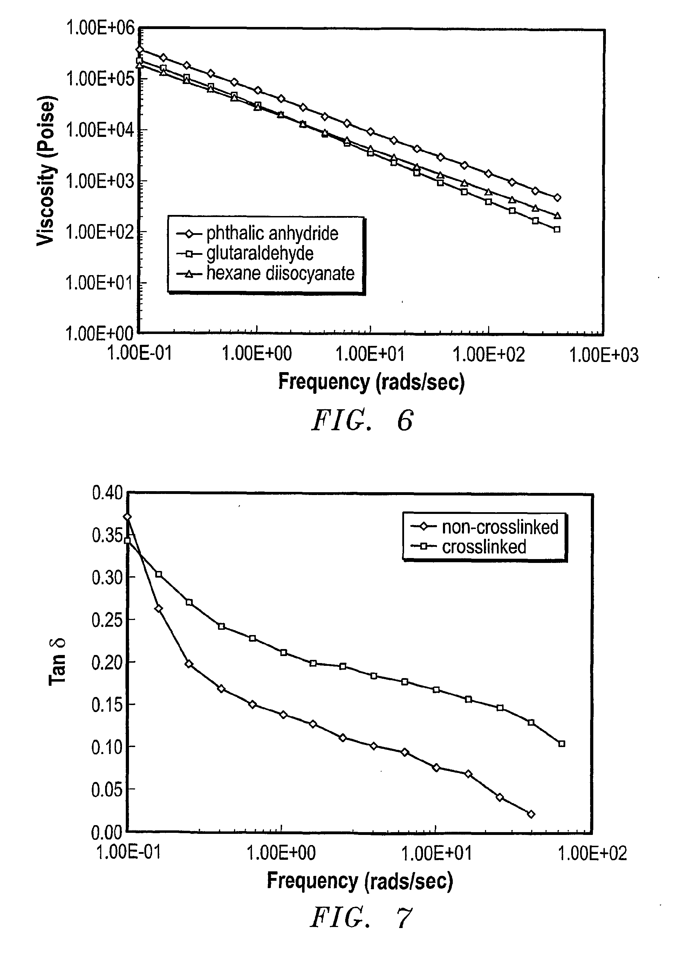

This process was repeated using phthalic anhydride and glutaraldehyde as crosslinkers. Hexane diisocyanate was also used as a crosslinker, but was added directly to the hydrogel as it was forming. The viscosities of the 4.8 wt. % hydrogels for each of these crosslinked systems are shown Fig. 6.

The impact of the crosslinking reaction was evaluated by comparison of the tan δ (ratio of the shear loss modulus to the shear storage modulus) for two of the gels, the uncrosslinked and the DER 332 resin-crosslinked gels. An overlay of tan δ for the 4.8 wt. % gels are shown in Fig. 7. The flattening tan δ curve of the DER 332 resin-crosslinked hydrogel suggests that this gel is more rigid than the uncrosslinked hydrogel.

The hydrogels subjected to contact with buffer solution at body temperature were checked periodically for degradation via viscosity measurements and UV-Vis spectroscopy of the buffer layer. After more than 6 weeks, no signs of degradation were present in any of the hydrogels.

### Example 6

Reduced, HMWK was prepared by placing 40 g of clean, dry human hair into a 1000 mL wide mouth glass reactor. 800 mL of a 0.8M solution of thioglycolic acid at pH 10.2 (adjusted with potassium hydroxide) was added and the mixture was stirred at room temperature under a nitrogen atmosphere for 18 hours. The solution was filtered and the liquid discarded. The hair was rinsed with copious amounts of deionized (DI) water, then placed back into the reactor. 400 mL of a 7M urea solution was added and the mixture was stirred at room temperature under a nitrogen atmosphere for 24 hours. After urea extraction, the solids were separated from the liquid by centrifugation. The liquid was added dropwise to a 10-fold volume excess of ethanol, thereby forming a keratin precipitate. The precipitated keratins were isolated by filtration and dried under vacuum. The resulting HMWK powder was ground by hand using a mortar and pestle.

### Example 7

500 g of clean, dry human hair was placed in a 12000 mL round bottom flask. 8350 mL of 1 weight/volume percent of hydrogen peroxide was added and the reaction heated to reflux for 180 minutes. The hair was separated from the liquid by filtration and the liquid discarded. The hair was rinsed with copious amounts of water and allowed to air dry. 100 g of the dried, oxidized hair was placed in a 2000 mL round bottom flask. 1000 mL of 1M thioglycolic acid at pH 9 (adjusted with ammonium hydroxide) was added and the mixture was heated to 60°C under a nitrogen atmosphere for 24 hours. After reductive extraction, the solids were separated from the liquid by centrifugation. The liquid was added dropwise to a 8-fold volume excess of ethanol, thereby forming a keratin precipitate. The precipitated HMWK keratins were isolated by filtration and dried under vacuum.

A solution was prepared by mixing 2 g of HMWK with 1 mL of 30% ammonium hydroxide and 10 mL of dimethyl sulfoxide. The solution was stirred and heated to ca. 75°C to effect dissolution of the keratins. The solution was split into 4 volumes and placed into separate vials. To each of these 4 solutions was added 5, 10, 15, and 20 weight percent (relative to HMWK) of hexanediisocyanate, respectively, and 0.05, 0.1, 0.15, and 0.2 weight percent (relative to HMWK) of butyldilauryltin catalyst, respectively. The solutions were mixed using a vortex mixer, poured into separate petri dishes, and placed under a heat lamp. After 120 minutes of exposure, the samples were removed from the heat and peeled from the petri dishes. After curing, a small piece of each elastomer was immersed in a pH 7 aqueous buffer solution. After exposure to aqueous buffer for 48 hours, elastomers made with 5, 15, and 20 weight percent hexanediisocyanate were unchanged. The sample made with 10 weight percent hexanediisocyanate began to slowly hydrolyze after only 24 hours.

### Example 8

4.5 g of the HMWK sample from Example 7 was dissolved in 2.25 mL of 30% ammonium hydroxide and 22.5 mL of dimethyl sulfoxide by heating to ca. 75°C and stirred. The solution was split into 9 separate vials and used to prepare the solutions described in the following Table.

| Sample | Terephthalic Acid (grams) | Phthalic Anhydride (grams) | DER™ 332 Resin (grams) | Sodium Acetate Catalyst (grams) |
|---|---|---|---|---|
| 1a (control) | - | - | - | - |
| 2a | 0.025 | - | - | 0.005 |
| 2b | 0.050 | - | - | 0.005 |
| 3a | - | 0.025 | - | 0.005 |
| 3b | - | 0.050 | - | 0.005 |
| 4a | - | - | 0.025 | - |
| 4b | - | - | 0.050 | - |

The solutions were poured into separate petri dishes and placed under a heat lamp for ca. 240 minutes. After curing, a small piece of each elastomer was immersed in a pH 7 aqueous buffer solution. After exposure to aqueous buffer for 48 hours, elastomers 1a, 2a, 2b, 3a, and 3b had disintegrated and partially dissolved. After 6 days, elastomers 4a and 4b remained intact.

### Example 9

175 grams of clean, dry human hair were extracted for 12 hours at room temperature with a solution of 265.9 grams of thioglycolic acid (TGA) diluted to 3.5 liters with deionized (DI) water. The pH of this solution was adjusted with 323 grams of potassium hydroxide (KOH) prior to use (pH 10.2). The extraction was performed with stirring under positive nitrogen pressure in a 4 liter glass reactor.

After extraction, the hair was separated by centrifugation followed by filtration and the liquid discarded. The reductively modified hair was rinsed with DI water to remove residual TGA and KOH then extracted with 3.2 liters of 7 molar aqueous urea solution. After 24 hours at room temperature, centrifugation and filtration isolated the extract. The aqueous protein solution was neutralized by addition of ca. 5 mL of hydrochloric acid and added to a 10-fold excess of ethanol. The resulting precipitate was rinsed with the following solutions:
1) 500 mL of 10/90 DI water/ethanol
2) 500 mL of ethanol
3) 500 mL of methanol
4) 500 mL of ethanol
The dehydrated keratin precipitate was place under vacuum to remove residual solvent, then ground into a fine powder.

4.0 grams of this keratin powder were mixed with a solution containing 0.4 grams of DER 736 resin (Aldrich Chemical Co., Milwaukee, WI), 0.5 grams of ethanol, and 75.1 grams of DI water. The ethanol was first used to dissolve the DER 736 resin, then added to the water. A thick suspension formed within 30 seconds.

This keratin suspension was poured into a 7 x 12 x 0.5 cm mold on a Teflon-lined glass plate. Over the hydrogel was placed a modified Telfa® pad (Beiersdorf Inc., Wilton, CT). The pad had been modified by removing the inner polymer liner, thereby exposing the inner cotton core. The pad was placed on the hydrogel with the cotton side down so that the gel would penetrate and bond to it. The outer polymer film of the pad would serve as a semipermeable barrier backing to the hydrogel dressing.

The mold was placed in an incubator at 37.5°C for 24 hours. After curing, the dressing was peeled from the Teflon mold and remained intact. Digital photographs of the resulting dressing are shown below.

The hydrogel can be cured onto any adherent polymer or natural fiber backing. The intent of the backing is to provide structure so that the dressing can be easily handled, but also to serve as a barrier to moisture and pathogens. Many types of films are used for this purpose and the choice of backing can be made such that an optimal level of moisture retention and protection can be achieved. The level of hydration of the keratin hydrogel can also be varied to provide absorption of wound exudate. The keratin hydrogel is also capable of delivering biologically active compounds, inherent in keratins prepared from hair using the process described, thereby accelerating the wound healing and skin remodeling cascade. The keratin hydrogel can also be loaded with drugs, cells, and/or other proteins to be delivered to the wound bed.

### WOUND STUDIES

### Example 10

175 grams of clean, dry human hair were extracted for 12 hours at room temperature with a solution of 265.9 grams of thioglycolic acid (TGA) diluted to 3.5 liters with deionized (DI) water. The pH of this solution was adjusted with 323 grams of potassium hydroxide (KOH) prior to use (pH 7.2). The extraction was performed with stirring under positive nitrogen pressure in a 4 liter glass reactor.

After extraction, the hair was separated by centrifugation followed by filtration and the liquid discarded. The reductively modified hair was rinsed with DI water to remove residual TGA and KOH then extracted with 3.2 liters of 7 molar aqueous urea solution. After 24 hours at room temperature, centrifugation and filtration isolated the extract. The aqueous protein solution was neutralized by addition of ca. 5 mL of hydrochloric acid and added to a 10-fold excess of ethanol. The resulting precipitate was rinsed with the following solutions:
5) 500 mL of 10/90 DI water/ethanol
6) 500 mL of ethanol
7) 500 mL of methanol
8) 500 mL of ethanol
The dehydrated keratin precipitate was place under vacuum to remove residual solvent, then ground into a fine powder. The keratin hydrogel was formed by adding 95.9 grams of ultrapure water to 4.01 grams of this powder (4 weight % solids) and mixing thoroughly.

### Example 11

The keratin powder from Example 10 was utilized to make this hydrogel with one exception. The ultrapure water used to form the hydrogel contained 0.1 weight percent of low molecular weight keratins. In so doing, the hydrogel was "spiked" with matrix proteins that normally would not be present in large quantities.

### Example 12

380 grams of clean, dry oxidized human hair were extracted for 24 hours at room temperature with a solution of 332.01 grams of TGA diluted to 3.46 liters with deionized (DI) water. The pH of this solution was adjusted to 9 with 400 mL of ammonium hydroxide (MH₄OH) prior to use). The extraction was performed with stirring under positive nitrogen pressure at 60°C in a 4-liter glass reactor fitted with a cold water condenser.

After extraction, the hair was separated by centrifugation followed by filtration. The aqueous protein solution was added to a 10-fold excess of ethanol. The resulting precipitate was rinsed with excess ethanol and dried under vacuum, then ground into a fine powder.

A keratin-silicone elastomer was formed by dissolving 15 grams of this keratin powder into 45 grams of DI water. A photoinitiator was added (0.3 grams of anthraquinone-2-sulfonic acid sodium salt monohydrate) and a solution of 3 grams of vinyl-terminated silicone (catalogue no. DMS-V03; Gelest, Inc., Tullytown, PA) in 2 grams of isopropyl alcohol. After ca. 20 minutes of stirring, the thick solution was cast onto a Teflon coated glass plate and cured under a UV lamp for ca. 4 hours. The film was then dried under a heat lamp for ca. 1 hour. Samples of this film were cut into 1" x 1" squares to be used as wound dressings.

### Example 13

380 grams of clean, dry oxidized human hair were extracted for 24 hours at room temperature with a solution of 332.01 grams of TGA diluted to 3.46 liters with deionized (DI) water. The pH of this solution was adjusted to 9 with 400 mL of ammonium hydroxide (NH₄OH) prior to use). The extraction was performed with stirring under positive nitrogen pressure at 60°C in a 4-liter glass reactor fitted with a cold water condenser.

After extraction, the hair was separated by centrifugation followed by filtration. The aqueous protein solution was added to a 10-fold excess of ethanol. The resulting precipitate was rinsed with excess ethanol and dried under vacuum, then ground into a fine powder.

20 grams of this keratin powder was dissolved in 60 grams of DI water, then added 0.2 grams of DER 736 resin (Aldrich, Milwaukee, WI) in 10 grams of isopropyl alcohol. The pH of the solution was adjusted to 7.0 by addition of 3 drops of NH₄OH. The solutions was stirred at room temperature for ca. 45 minutes, then poured into a mold on Teflon coated glass. The film was cured using slight heating for ca. 7 hours. Samples of this film were cut into 1" x 1" squares to be used as wound dressings.

### Example 14

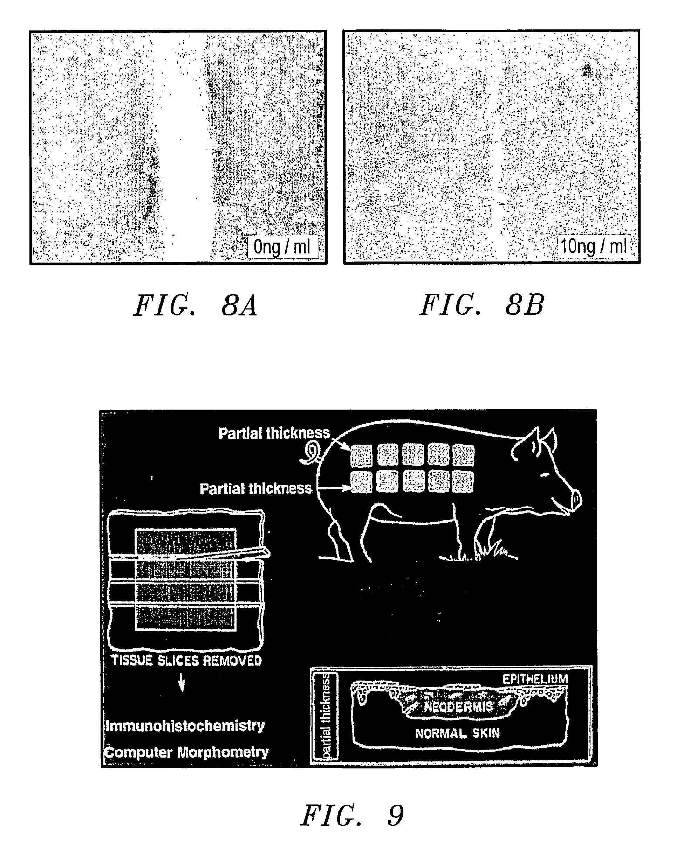

Physiologic stimulation with human hair extracts and extracts from keratin biomaterials were used to establish evidence of biological responses. Second or third passage cells were grown in serum containing media to ∼80% confluence on fibronectin-coated Nunc single chamber slides. Cells were serum starved for 6 hours and then 6 wounds (scratch assays; see Figure 8) were created per slide. Defined media (minus serum) with and without keratins were introduced, and cells were returned to the incubator to heal for 18 to 48 hours followed by fixation in 4% paraformaldehyde and staining with hematoxylin. During this healing interval, cells attempt to close the defect with mitosis and migration.

An initial dose response series was employed to screen for effective and deleterious concentrations of keratins. The size of the wounded area was assessed using Image Proplus software (Media Cybernetics) and comparisons were made to determine the amount of healing.

Fibroblast cultures showed biological activation by some of the keratin solutions. Increases in healing as high as 230% over the control were measured. Concentrations of keratin as low as 0.001 mg/mL showed statistically significant (p<0.05) increases in healing.

A-431 cell cultures (a mouse epithelial cell line) showed biological activation by most of the keratin solutions. Increases in healing as high as 230% over the control were measured. Concentrations of keratin as low as 0.001 mg/mL showed statistically significant (p<0.05) increases in healing.

### Example 15

A Yorkshire pig (*Sus scrofa*) weighing approximately 34.00-45.36 kg (75-100 lbs) was used. Partial-thickness excisions (open wound beds) were created with a Padgett dermatome calibrated to shave a thickness of ca. 1200 µm (microns). Figure 1 illustrates wound placement patterns and depicts techniques used in this study to examine the histological specimens at different time points during wound repair.

The initial pig received a series of partial thickness excisions created with a Padgett Dermatome. The wounds that were destined to become the 7-day wounds (10 of these) were created on the initial day of the experiment. The wounds that were destined to become the 4-day wounds were created 3 days after the initiation of the study (10 of these). When the pig was euthanized at the conclusion of the experiment, all the wounds were removed for analysis. Formulations were applied to each wound on a daily basis. Wounds were then wrapped in semi-occlusive bandage (Op-site) to ensure that the formulation remained in position and to prevent mixing of the various topical formulations. The semi-occlusive bandage also acted to facilitate healing by maintaining a moist environment. Wound treatments were as follows: control (only occlusive dressing), Gel 4-AKR-178-2 (Example 10), Gel 4-AKR-178-12 (Example 11), Film 1-SEB-113-2 (Example 13), Film 7-AR-44-2 (Example 12). In this initial study all wound treatments were performed in duplicate. Thus the number of wounds were two per day per treatment group.

Quantitative outcomes included epidermal resurfacing, the influx (cell density) of neutrophils or monocyte/macrophages, and capillary density. Morphological differences between the various dosages and compounds, if present, were detected by a number of routine and special stains performed on histological specimens. The wounds were stained with Gomori's trichrome which is useful for delineating the wound margins. The proliferating cells (keratinocytes or fibroblasts) were identified using specific immunomarkers (PCNA - proliferating cell nuclear antigen). The density of neutrophils and monocytes and macrophages were quantified manually using standard morphological indicators such as nuclear morphology and size differentials.

The status of the vascular network within wounds is important for several reasons. During the acute wound healing period, endothelial cells can potentially regulate the inflammatory response by controlling the transmigration of neutrophils and monocytes into the wound bed. In addition, angiogenesis is a reliable indicator of the healing kinetics within a wound bed. If wounds mature normally, capillary density diminishes. If warranted, immunohistological evaluations were performed retrospectively when tissues were removed. Capillary density was assessed using selective immunostaining of Factor VIII+ endothelial cells followed by computerized quantitative morphometric analysis. Wounds were collected to evaluate for short-term effects such as proliferation, migration of epidermal cells, fibroblasts and endothelial cells.

### Histological Impressions

The most notable feature histologically was the hypertrophic response of the epidermis for both of the gels and for both of the films. This finding was consistently observed in all of the sections except for the controls. It definitely appeared that there were bioactive ingredients in each of the 4 formulations, causing a gross biological outcome change. This feature was most prominent in the 7-day wounds. While all porcine wounds were 100% resurfaced at this timepoint, the treatments exhibited their greatest impact on the morphological appearance of the dermal-epidermal junction. In control wounds the dermal-epidermal junction was largely flat and unremarkable as it is in typical scars. By contrast, in the test wounds, there were numerous downward and upward projections of the epidermal rete ridges and the numbers of epidermal layers appeared mostly increased. This suggests that the newly healed epidermis in the test wounds would be more likely to adhere to the surface of the dermis and would be less likely to shear off and be as fragile as normal. These results indicate that keratinocytes of the epidermis and epidermal appendages are especially responsive to certain cytokines and mediators in these formulations.

### Quantitative Morphometric Analysis:

**Re-Epithelialization:** At 4 days, several of the wounds appeared remarkably more resurfaced than the control. The variance between the duplicate samples was widely divergent, probably due to some variations in the original thickness of the wounds. However, when taken with the histological evidence, we can say with confidence that the formulations have had an affect on the epidermis. At 7 days essentially all the wounds are 100% resurfaced as would be expected.

**Dermal Depth:** At day 4, the control samples were not measurable so we had no true basis for comparison. From the day 7 material, the two duplicate control samples were very close in depth and suggest a reasonable baseline for comparison.

The following table summarizes the epithelial resurfacing data:

| **Treatment Group** | **Average % Healed** |
|---|---|
| Hydrogel A | 39.80 |
| Hydrogel B | 16.45 |
| Elastomer A | 27.15 |
| Elastomer B | 25.40 |
| Control | 11.80 |

### Example 16

Procedure. The two pigs used in this study received a series of partial thickness excisions and full-thickness excisions created with a Padgett Dermatome. The wounds that were destined to become the 14-day wounds were created on the initial day of the experiment. The wounds that were destined to become the 7-day wounds were created 7 days after the initiation of the study. When the pig was euthanized at the conclusion of the experiment, all the wounds were removed for analysis. The right side of the pig was given the partial thickness wounds and the left side of the pig received the full thickness wounds. The pigs were given preanesthetics of atropine, ketamine and acepromazine. They were maintained on inhalation anesthesia of Nitrous oxide and oxygen. Postoperative medications included Buprenex every 12 hours for analgesia and cephelexin to prevent infection. Keratin formulations were applied to each wound on a daily basis for a period of 5 days of continuous treatment. Wounds were then wrapped in semi-occlusive bandage (Op-site) to ensure that the formulation remained in position and to prevent mixing among topical formulations and to prevent mechanical trauma to the healing wounds. The semi-occlusive bandage also acted to facilitate healing by maintaining a moist environment. Wound treatments were either the gel formulation or no treatment. In this initial study all wound treatments were performed in duplicate. Thus the numbers of wounds were two per day per treatment group per pig. N = 4 wounds in this experiment for each treatment.

**Porcine Observations:** The two pigs appeared to tolerate the formulation and the wounding quite well. There were no obvious signs of infection observed either grossly or was their evidence of infection observed in the histological sections. The semi-occlusive dressing remained firmly in place and no wounds were subjected to unexpected desiccation.

***Quantitative Morphometric Analysis:*** The quantitative morphometric analyses were performed using an Olympus AH2 light microscope that was interfaced to a Pixcera digital camera. Images in jpg format were collected and analyzed using Image Proplus software (Media Cybernetics). Analysis of resurfacing was performed on histological sections that were stained with Gomori's one-step Trichrome. Three random sections from each wound were positioned on glass slides. The total distance on one end of the wound to the other end is assessed in µm (microns), and then the distance of new epithelial coverage was added. This represents the sum of the epithelial lengths growing out from the wounds edges as well as the outgrowths from islands of new epithelium that emanate from epidermal remnants such as hair follicles and sweat ducts.

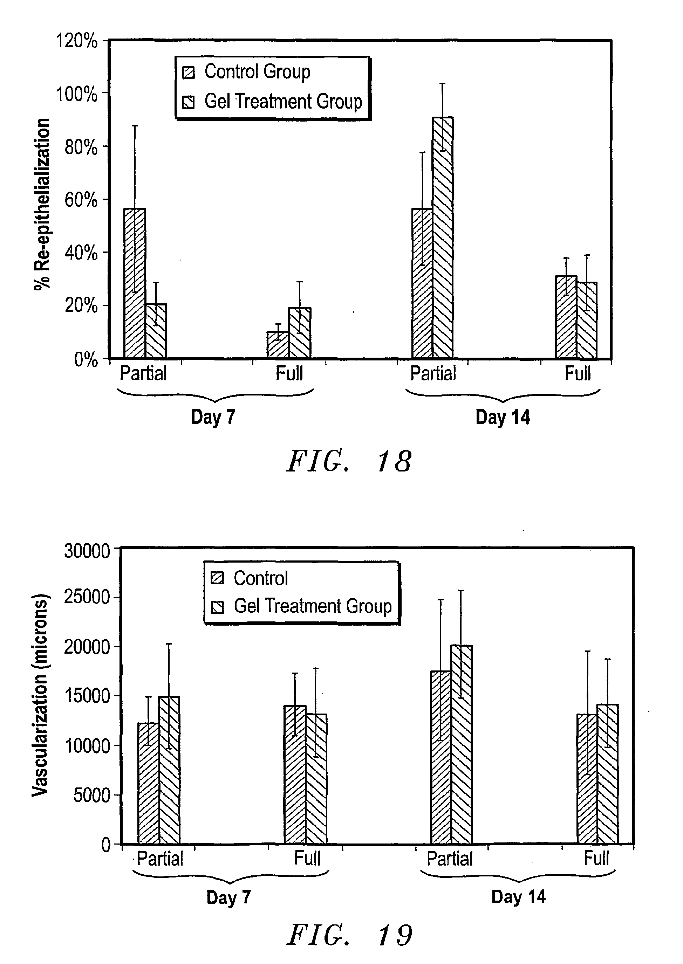

*Re-Epithelialization:* Some notable differences were observed among the 8 different treatment groups. In the partial thickness wounds after 7 days of healing, the wounds were better resurfaced in the control group as opposed to the gel treated group. However, when the experiment was allowed to proceed further and the tissue was harvested after 14 days, it was apparent that the mean resurfacing was 91% in the gel treatment and only 56% resurfaced in the control gel group. These data are summarized in the graph in Figure 1.
*Neovascularization:* Data from the 7-day treatment groups was fairly unremarkable. While the keratin gel in the partial treatment group appeared to show a trend toward a statistically significant difference between keratin treatment and the control wounds, it would require additional pigs to confirm whether this was indeed true or was merely the result of chance. In the wounds that were treated for 14 days, the keratin gel treatment appeared to stimulate a robust angiogenic response in the partial thickness group of wounds. This difference did not reach statistical significance in the two pigs that were studied (N = 4 wounds). These data are summarized in the graph in Figure 18. The keratin gel treatment was unremarkable in the full-thickness wounds due to the immaturity of these wounds.

## Claims

1. A tissue defect dressing comprising a keratinaceous salve comprising a hydrogel comprising water retained in a keratinaceous network comprising keratin molecules networked by interkeratin associations other than glutaraldehyde, the interkeratin associations comprising covalent bonds between the keratin molecules comprising primarily other than disulfide bonds, said interkeratin associations producing a keratinaceous network and
wherein the covalent bonds comprise primarily interkeratin crosslinks comprising first covalent bonds between first functional groups on a plurality of molecules of a crosslinking agent and first reactive pendant groups on a plurality of first water soluble keratins and second covalent bonds between second functional groups on a plurality of molecules of the crosslinking agent and second reactive pendant groups on a plurality of second water soluble keratins and
wherein the crosslinking agent is a heterogeneous crosslinking agent and wherein the heterogeneous crosslinking agent produces crosslinks comprising the following structure: 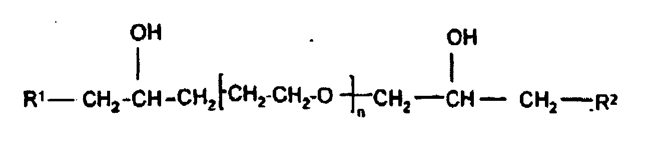 wherein
n is from 1 to 50; and,
R¹ and R² independently are amino acid residues of separate water soluble keratins, the residues being selected from the group consisting of cysteine, arginine, serine, lysine, asparagine, glutamine, tyrosine, tryptophan, and histidine.

2. The tissue defect dressing of claim 1 further comprising one or more added bioactive component.

3. The tissue defect dressing of any of claim 1 and 2 wherein the keratinaceous molecules are α-keratinaceous molecules.

4. The tissue defect dressing of any of claims 1-2 and 3 comprising a cohesive tissue defect dressing.

5. The tissue defect dressing of claim 4 further comprising a support adapted to support the proteinaceous salve.

6. The tissue defect dressing of claim 5 wherein the support is adapted to render the tissue defect dressing cohesive.

7. The tissue defect dressing of claim 6 wherein the support comprises a sheet of permeable material having an inner surface abutting the proteinaceous salve and an outer surface abutting a barrier material.

8. The tissue defect dressing of any of claims 1-6 and 7 wherein the keratin molecules are derived from human hair.

9. The tissue defect dressing of any of claims 1-7 and 8 wherein the covalent bonds consist essentially of interkeratin crosslinks comprising first covalent bonds between first functional groups on a plurality of molecules of a crosslinking agent and first reactive pendant groups on a plurality of first water soluble keratins and second covalent bonds between second functional groups on a plurality of molecules of said crosslinking agent and second reactive pendant groups on a plurality of second water soluble keratins.

10. The tissue defect dressing of any of claims 1-9 wherein the first and second functional groups comprise groups selected from the group consisting of alkoxide groups, allyl groups, vinyl groups, hydroxyl groups, amine groups, aldehyde groups, isocyanate groups, ester groups, and anhydride groups.

11. The tissue defect dressing of any of claims 1-10 wherein the reactive pendant groups comprise groups selected from the group consisting of hydroxyl groups, thiol groups, reactive amine groups, and epoxides.

12. The tissue defect dressing of any of claims 1-11 wherein at least one of a moiety selected from the group consisting of the first functional groups, the first reactive pendant groups, the second functional groups, and the second reactive pendant groups comprises an epoxide.

13. The tissue defect dressing of claims 1-12 wherein the heterogeneous crosslinking agent produces crosslinks comprising the following stru ctu re : 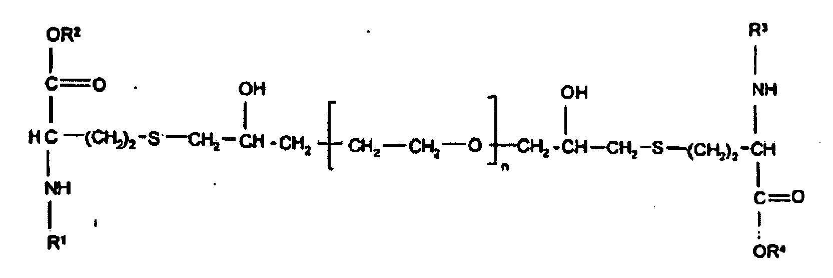 wherein
n is from 1 to 50;
R¹ and R² are the remainder of a first water soluble keratin; and,
R³ and R⁴ are the remainder of a second water soluble keratin.

## Patentansprüche

1. Gewebedefektverband, umfassend eine keratinöse Salbe umfassend ein Hydrogel mit retiniertem Wasser in einem keratinösen Netzwerk, welches Keratinmoleküle umfasst, die durch interkeratine Verbindungen vernetzt und nicht Glutaraldehyd sind, wobei die interkeratinen Verbindungen kovalente Bindungen zwischen den Keratinmolekülen umfassen, die hauptsächlich andere als Disulfidbindungen sind, wobei diese interkeratinen Verbindungen ein keratinöses Netzwerk erzeugen und
wobei die kovalenten Bindungen hauptsächlich interkeratine Kreuzvernetzungen umfassen, die erste kovalente Bindungen zwischen den ersten funktionalen Gruppen an einer Vielzahl an Molekülen eines Kreuzvernetzers und ersten reaktiven Pendantgruppen an einer Vielzahl an ersten wasserlöslichen Keratinen und zweite kovalente Bindungen zwischen den zweiten funktionalen Gruppen an einer Vielzahl an Molekülen des Kreuzvernetzers und zweiten reaktiven Pendantgruppen an einer Vielzahl an zweiten wasserlöslichen Keratinen aufweisen und
wobei der Kreuzvernetzer ein heterogener Kreuzvernetzer ist und der heterogene Kreuzvernetzer Kreuzvernetzungen erzeugt, die folgende Struktur umfassen: 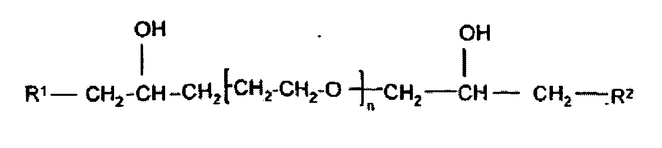 wobei
n 1 bis 50 ist; und
R¹ und R² unabhängig Aminosäurereste verschiedener wasserlöslicher Keratine sind, wobei die Reste ausgewählt sind aus der Gruppe bestehend aus Cystein, Arginin, Serin, Lysin, Asparagin, Glutamin, Tyrosin, Tryptophan und Histidin.

2. Gewebedefektverband nach Anspruch 1, der des Weiteren ein oder mehr hinzugefügte bioaktive Komponenten umfasst.

3. Gewebedefektverband nach einen der Ansprüche 1 oder 2, wobei die keratinösen Moleküle α-keratinöse Moleküle sind.

4. Gewebedefektverband nach einem der Ansprüche 1-2 und 3 umfassend einen kohäsiven Gewebedefektverband.

5. Gewebedefektverband nach Anspruch 4, der des Weiteren eine Stütze bzw. Auflage aufweist, um die proteinöse Salbe zu halten.

6. Gewebedefektverband nach Anspruch 5, wobei die Stütze bzw. Auflage angepasst ist, um den Gewebedefektverband kohäsiv zu gestalten.

7. Gewebedefektverband nach Anspruch 6, wobei die Stütze bzw. Auflage ein Blatt eines durchlässigen Materials aufweist, mit einer inneren Seite, die an die proteinöse Salbe grenzt und einer äußeren Seite, die an ein Barrierematerial grenzt.

8. Gewebedefektverband nach einem der Ansprüche 1-6 und 7, wobei die Keratinmoleküle aus menschlichem Haar stammen.

9. Gewebedefektverband nach einem der Ansprüche 1-7 und 8, wobei die kovalenten Bindungen im Wesentlichen aus interkeratinen Kreuzvernetzungen bestehen, die erste kovalente Bindungen zwischen ersten funktionalen Gruppen an einer Vielzahl an Molekülen eines Kreuzvernetzers und ersten reaktiven Pendantgruppen an einer Vielzahl an ersten wasserlöslichen Keratinen und zweite kovalente Bindungen zwischen zweiten funktionalen Gruppen an einer Vielzahl von Molekülen des Kreuzvernetzers und zweiten reaktiven Pendantgruppen an einer Vielzahl an zweiten wasserlöslichen Keratinen umfassen.

10. Gewebedefektverband nach einem der Ansprüche 1-9, wobei die ersten und zweiten funktionalen Gruppen Gruppen aufweisen, die ausgewählt sind aus der Gruppe bestehend aus Alkoxidgruppen, Allylgruppen, Vinylgruppen, Hydroxylgruppen, Amingruppen, Aldehydgruppen, Isocyanatgruppen, Estergruppen, und Anhydridgruppen.

11. Gewebedefektverband nach einem der Ansprüche 1-10, wobei die reaktiven Pendantgruppen Gruppen aufweisen, die ausgewählt sind aus der Gruppe bestehend aus Hydroxylgruppen, Thiolgruppen, reaktiven Amingruppen und Epoxiden.

12. Gewebedefektverband nach einem der Ansprüche 1-11, wobei mindestens einer der Reste, ausgewählt aus der Gruppe bestehend aus den ersten funktionalen Gruppen, den ersten reaktiven Pendantgruppen, den zweiten funktionalen Gruppen und den zweiten reaktiven Pendantgruppen ein Epoxid umfasst.

13. Gewebedefektverband nach einem der Ansprüche 1-12, wobei der heterogene Kreuzvernetzer Kreuzvernetzungen erzeugt, die folgende Struktur umfassen: 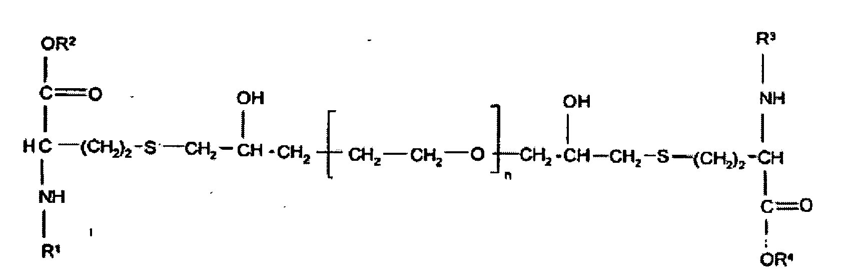 wobei
n 1 bis 50 ist;
R¹ und R² die Reste eines ersten wasserlöslichen Keratins sind; und
R³ und R⁴ die Reste eines zweiten wasserlöslichen Keratins sind.

## Revendications

1. Pansement pour anomalie tissulaire comprenant un baume kératine comprenant un hydrogel comprenant de l'eau retenue dans un réseau kératineux comprenant des molécules de kératine réseautées par des associations d'interkératine autres que le glutaraldéhyde, les associations d'interkératine comprenant des liaisons covalentes entre les molécules de kératine comprenant principalement des liaisons autres que des liaisons disulfure, lesdites associations d'interkératine produisant un réseau kératineux et
où les liaisons covalentes comprennent principalement des réticulations d'interkératine comprenant des premières liaisons covalentes entre des premiers groupes fonctionnels sur une pluralité de molécules d'un agent de réticulation et des premiers groupes pendants réactifs sur une pluralité de premières kératines solubles dans l'eau et des secondes liaisons covalentes entre des seconds groupes fonctionnels sur une pluralité de molécules de l'agent de réticulation et des seconds groupes pendants réactifs sur une pluralité de secondes kératines solubles dans l'eau et
où l'agent de réticulation est un agent de réticulation hétérogène et où l'agent de réticulation hétérogène produit des réticulations comprenant la structure suivante : 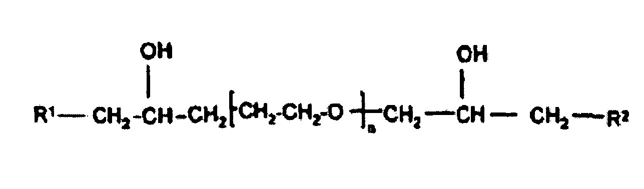 où
n vaut de 1 à 50 ; et
R¹ et R² sont indépendamment des résidus d'acides aminés de kératine soluble dans l'eau séparées, les résidus étant choisis dans le groupe consistant en la cystéine, l'arginine, la sérine, la lysine, l'asparagine, la glutamine, la tyrosine, le tryptophane et l'histidine.

2. Pansement pour anomalie tissulaire selon la revendication 1, comprenant en outre un ou plusieurs composants bioactifs ajoutés.

3. Pansement pour anomalie tissulaire selon l'une quelconque des revendications 1 et 2, dans lequel les molécules kératineuses sont des molécules α-kératineuses.

4. Pansement pour anomalie tissulaire selon l'une quelconque des revendications 1, 2 et 3, comprenant un pansement pour anomalie tissulaire cohésif.

5. Pansement pour anomalie tissulaire selon la revendication 4, comprenant en outre un support adapté pour supporter le baume protéique.

6. Pansement pour anomalie tissulaire selon la revendication 5, dans lequel le support est adapté pour rendre cohésif le pansement pour anomalie tissulaire.

7. Pansement pour anomalie tissulaire selon la revendication 6, dans lequel le support comprend une feuille de matériau perméable ayant une surface interne en butée contre le baume protéique et une surface externe en butée contre un matériau formant barrière.

8. Pansement pour anomalie tissulaire selon l'une quelconque des revendications 1 à 6 et 7, dans lequel les molécules de kératines sont dérivées de cheveux ou poils humains.

9. Pansement pour anomalie tissulaire selon l'une quelconque des revendications 1 à 7 et 8, dans lequel les liaisons covalentes consistent essentiellement en des réticulations d'interkératine comprenant des premières liaisons covalentes entre des premiers groupes fonctionnels sur une pluralité de molécules d'un agent de réticulation et des premiers groupes pendants réactifs sur une pluralité de premières kératines solubles dans l'eau et des secondes liaisons covalentes entre des seconds groupes fonctionnels sur une pluralité de molécules dudit agent de réticulation et des seconds groupes pendants réactifs sur une pluralité de secondes kératines solubles dans l'eau.

10. Pansement pour anomalie tissulaire selon l'une quelconque des revendications 1 à 9, dans lequel les premiers et seconds groupes fonctionnels comprennent des groupes choisis dans le groupe consistant en les groupes alcoxydes, les groupes allyle, les groupes vinyle, les groupes hydroxyle, les groupes amine, les groupes aldéhyde, les groupes isocyanate, les groupes ester, les groupes anhydride.

11. Pansement pour anomalie tissulaire selon l'une quelconque des revendications 1 à 10, dans lequel les groupes pendants réactifs comprennent des groupes choisis dans le groupe consistant en les groupes hydroxyle, les groupes thiol, les groupes amine réactive, et les époxydes.

12. Pansement pour anomalie tissulaire selon l'une quelconque des revendications 1 à 11, dans lequel au moins un élément parmi un fragment choisi dans le groupe consistant en les premiers groupes fonctionnels, les premiers groupes pendants réactifs, les seconds groupes fonctionnels et les seconds groupes pendants réactifs, comprend un époxyde.

13. Pansement pour anomalie tissulaire selon les revendications 1 à 12, dans lequel l'agent de réticulation hétérogène produit des réticulations comprenant la structure suivante : 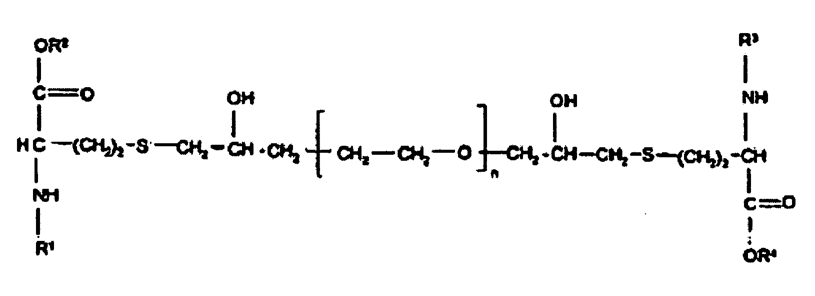 où
n vaut de 1 à 50 ;
R¹ et R² sont le reste d'une première kératine soluble dans l'eau ; et
R³ et R⁴ sont le reste d'une seconde kératine soluble dans l'eau.
